# EUROPEAN PATENT SPECIFICATION

(11) **EP 2 509 607 B1**
(45) Date of publication and mention of the grant of the patent: **06.09.2017**
(21) Application number: 10836295.5
(22) Date of filing: 08.12.2010
(51) Int. Cl.: A61K 35/32, C12N 5/077, A61K 31/726, A61P 19/00, A61P 19/02, A61P 19/08

(54) **GLYCOSAMINOGLYCAN MIXTURES**
GLYCOSAMINOGLYCANMISCHUNGEN
MÉLANGES DE GLYCOSAMINOGLYCANES

(30) Priority: 09.12.2009 US 267866 P
(43) Date of publication of application: 17.10.2012
(73) Proprietor: Agency for Science, Technology and Research, Singapore 138632 (SG)
(72) Inventor: COOL, Simon, Singapore 138648 (SG); NURCOMBE, Victor, Singapore 138648 (SG)
(74) Representative: Clegg, Richard Ian
(86) International application number: PCT/SG2010/000456
(87) International publication number: WO 2011/071453

(56) References cited:
- WO-A1-2010/030244
- US-B2- 6 919 312
- FEDARKO N S ET AL: "High-performance liquid chromatographic separation of hyaluronan and four proteoglycans produced by human bone cell cultures", ANALYTICAL BIOCHEMISTRY, ACADEMIC PRESS INC, NEW YORK, vol. 188, no. 2, 1 August 1990 (1990-08-01), pages 398-407, XP024819681, ISSN: 0003-2697, DOI: 10.1016/0003-2697(90)90627-L [retrieved on 1990-08-01]
- GRUENERT MARTIN ET AL: "Isolation of a native osteoblast matrix with a specific affinity for BMP2", JOURNAL OF MOLECULAR HISTOLOGY, vol. 38, no. 5, October 2007 (2007-10), pages 393-404, XP002695542, ISSN: 1567-2379
- BERESFORD J N ET AL: "Analysis of the Proteoglycans Synthesized by Human Bone Cells in Vitro", JOURNAL OF BIOLOGICAL CHEMISTRY, AMERICAN SOCIETY FOR BIOCHEMISTRY AND MOLECULAR BIOLOGY, US, vol. 262, no. 35, 15 December 1987 (1987-12-15), pages 17164-17172, XP003013501, ISSN: 0021-9258
- DATABASE WPI Week 200828 Thomson Scientific, London, GB; AN 2008-D92703 XP002695543, & JP 2008 074732 A (SEIKAGAKU KOGYO CO LTD) 3 April 2008 (2008-04-03)
- DATABASE WPI Week 200455 Thomson Scientific, London, GB; AN 2004-565169 XP002695544, & JP 2004 210715 A (SEIKAGAKU KOGYO CO LTD) 29 July 2004 (2004-07-29)
- JACKSON R. ET AL: 'Heparan Sulfate Regulates the Anabolic Activity of MC3T3-E1 Preosteoblast Cells by Induction of Runx2' JOURNAL OF CELLULAR PHYSIOLOGY vol. 210, 2007, pages 38 - 50, XP008159248
- SHINMYOUZU K. ET AL: 'Dermatan Sulfate Inhibits Osteoclast Formation by Binding to Receptor Activator of NF-kappaB Ligand' BIOCHEMICAL AND BIOPHYSICAL RESEARCH COMMUNICATIONS vol. 354, 2007, pages 447 - 452., XP008159277
- BAUD'HUIN M. ET AL: 'Glycosaminoglycans Inhibit Rankl-Induced Osteoclastogenesis. (P214)' BONE vol. 44, June 2009, page S334, XP008159246
- HUNTER G. ET AL: 'Isolation of Three Species of Proteoglycan Synthesized by Cloned Bone Cells' BIOCHEMISTRY vol. 22, 1983, pages 831 - 837, XP008159280
- LING L. ET AL: 'Glycosaminoglycans Modulate RANKL-Induced Osteoclastogenesis' JOURNAL OF CELLULAR BIOCHEMISTRY vol. 109, no. 6, April 2010, pages 1222 - 1231, XP008159308

## Description

### Field of the Invention

The present invention relates to osteoblast derived glycosaminoglycan mixtures and to their use in one or more of: inhibition of osteoclastogenesis, enhancement of proliferation of osteoblasts, and/or the treatment or prevention of bone fracture or bone deterioration.

### Background to the Invention

Bone remodeling is a coordinated process involving a balance between bone matrix synthesis by osteoblasts and bone resorption by osteoclasts. This constant process is essential for the maintenance of skeletal integrity [Boyle et al., 2003; Karsenty and Wagner, 2002; Roodman, 1999; Theill et al., 2002]. Multinucleated osteoclasts originate from monocyte / macrophage cells in bone marrow from the hematopoietic lineage [Suda et al., 1992]. Increased osteoclast activity has been observed in many osteopenic disorders, including osteoporosis, lytic bone metastases or rheumatoid arthritis, and ultimately results in abnormal bone resorption and bone fractures [Roodman, 1999]. Among the numerous factors affecting osteoclastogenesis, the receptor activator of NF-κB (RANK), its ligand RANKL and osteoprotegerin (OPG), the decoy receptor of RANKL, are essential for osteoclast development [Anderson et al., 1997; Wong et al., 1997; Yasuda et al., 1998].

RANKL is a TNF (tumor necrosis factor) ligand superfamily member [Anderson et al., 1997; Lacey et al., 1998; Wong et al., 1997; Yasuda et al., 1998]. It is produced by osteoblasts and bone marrow stromal cells whereas its receptor RANK is expressed on the surface of preosteoclasts [Fuller et al., 1998]. The interaction between RANKL and RANK stimulates the maturation of targeted osteoclasts by activation of transcription factors that regulate osteoclastogenesis [Blair et al., 2007; Burgess et al., 1999; Hsu et al., 1999; Jimi et al., 1999; Lacey et al., 1998; Matsuzaki et al., 1998]. RANKL is also required for the survival of mature osteoclasts [Katagiri and Takahashi, 2002]. OPG is produced by osteoblasts and acts as a decoy receptor to RANKL, thus inhibiting osteoclast differentiation and survival through competing with RANK for RANKL binding [Bolon et al., 2001; Simonet et al., 1997; Yasuda et al., 1998].

Evidence from genetic studies has shown that RANKL and RANK are essential for osteoclastogenesis. *RANK* or *RANKL* deficient mice exhibit marked osteopetrosis and a defect in tooth eruption caused by the diminishment of osteoclast formation [Dougall et al., 1999; Kim et al., 2000; Kong et al., 1999; Li et al., 2000]. Whereas mice lacking OPG have increased number and activity of osteoclasts and consequently suffer from severe osteoporosis [Bucay et al., 1998; Mizuno et al., 1998].

Accumulating clinical evidence has further consolidated the key role of the RANKL/RANK/OPG system in bone metabolism and their direct relevance to human disease. In several bone metabolic diseases, including postmenopausal osteoporosis, glucocorticoid-induced osteoporosis, multiple myeloma associated osteolytic lesions and atherosclerotic disease associated vascular calcification, an elevation of RANKL and/or a decline of OPG has been demonstrated. This imbalance has been further implicated as the key mechanism responsible for osteoporosis [Eghbali-Fatourechi et al., 2003; Pearse et al., 2001; Sasaki et al., 2002; Schoppet et al., 2004]. Furthermore, inactivating mutations in the OPG gene and activating mutations in the RANK gene have been identified in several rare genetic disorders of mineral metabolism [Hughes et al., 2000; Nakatsuka et al., 2003; Whyte and Hughes, 2002; Whyte et al., 2002]. All of these genetic abnormalities result in unopposed activation of RANK/RANKL signaling, which enhances osteoclastogenesis and consequently increases bone loss.

Recent studies have highlighted an important role of glycosaminoglycans (GAG) in the interaction and activity of RANK/RANKL. GAGs are polyanionic linear polysaccharides composed of repeating disaccharide units with a carboxyl group and one or more sulfates [Lamoureux et al., 2007]. Most GAGs are covalently attached to core proteins to form proteoglycans which are the major components of bone extracellular matrix [lozzo, 1998]. Endogenous GAGs include heparin, heparan sulfate, chondroitin sulfate, dermatan sulfate, keratin sulfate and hyaluronic acid. GAGs regulate a wide variety of biological processes, e.g. hemostasis, inflammation, angiogenesis, cytokine presentation/binding, cell adhesion and migration as well as the control of proliferation and differentiation [Gandhi and Mancera, 2008; Perrimon and Bernfield, 2000]. GAGs bind to a large number of protein ligands via interaction with protein heparin-binding domains thereby modifying a proteins biological activity. Recent studies have demonstrated that dermatan sulfate and heparin possessed high affinity to RANKL and suppress osteoclast formation via obstructing the interaction between RANKL and RANK [Ariyoshi et al., 2008; Shinmyouzu et al., 2007].

Since osteoclastogenesis is largely controlled by factors produced by osteoblasts (ie RANKL), it is important to understand how osteoblast-specific GAGs regulate osteoclast formation via interactions with RANKL. GAGs synthesized and secreted by osteoblasts are attached to the cell surface and contained within the extracellular matrix as a mixture of species with varying structure and activity [Haupt et al., 2009; Jackson et al., 2007]. The integrity of GAGs are important to maintain the proliferation and differentiation of osteoblasts [Kumarasuriyar et al., 2009]. Furthermore, bone derived heparan sulfates promote the proliferation and differentiation of osteoblasts and their activity is fine-tuned by structural changes at different developmental stages during osteogenesis [Haupt et al., 2009; Jackson et al., 2007; Nurcombe et al., 2007]. This mixture of GAGs may help regulate osteoclastogenesis in microenvironments where osteoblasts/osteoclasts co-reside.

Ariyoshi et al (Heparin Inhibits Osteoclastic Differentiation and Function. Journal of Cellular Biochemistry 103:1707-1717 (2008)) looked at the effects of chondroiitin sulphate species (not mixtures) and isolated heparin on mouse monocytic RAW 264.7 cells. They report that heparin inhibits osteoclast differentiation and that heparin binds to RANKL. They concluded that such binding ability is involved in the inhibition of osteoclast formation and function. They also reported that heparin suppressed TRAP-positive multinucleated cell formation and TRAP activity induced by RANKL, whereas the other GAGs tested showed no effects on osteoclast differentiation. Heparin also inhibited the formation of resorption pits, while the other GAGs tested did not. They concluded that the inhibitory effect of heparin toward osteoclastogenesis induced by RANKL is due to the binding of heparin to RANKL.

Atsushi Irie et al (Heparin enhances osteoclastic bone resorption by inhibiting osteoprotegrin activity. Bone 41 (2007) 165-174) reported that heparin does not bind to RANK or RANKL suggesting that heparin does not interact directly with either of these proteins. Instead, they report heparin to specifically bind to osteoprotegrin (OPG) and prevent OPG-mediated inhibition of osteoclastic bone resorption in co-culture. They concluded that heparin is a strong inducer of osteoclastic activation, enhancing osteoclastic bone resorption by a mechanism of inhibiting OPG activity, not RANKL. They also report that OPG binds heparin but not chondroitin sulphates or keratin sulphates.

Ariyoshi et al (Mechanisms Involved in Enhancement of Ostoeclast Formation and Function by Low Molecular Weight Hyaluronic Acid. The Journal of Biological Chemistry. Vol. 280, No. 19, May 13 pp18967-18972, 2005) indicates that low molecular weight hyaluronic acid enhances pit formation (i.e. bone resoprtion) whereas high molecular weight hyaluronic acid does not. JP2008-074732 describes an agent for treating an osteal defect, which is an heparan sulphate or heparin that increases BMP activity. The heparan sulfate or heparin is to be administered in conjunction with BMP2.

Jackson et al., (Journal of Cellular Physiology 210:38-50 (2007) describes heparan sulphates harvested from differentiating preosteoblast cells and their effect on growth and differentiation of preosteoblast cells.

### Summary of the Invention

In one aspect of the present disclosure a glycosaminoglycan mixture obtained from mammalian osteoblasts is provided. The glycosaminoglycan mixture may be in isolated or substantially purified form, e.g. wherein the glycosaminoglycan content of the composition is one of about 80%, 90%, 95% or more.

The osteoblasts from which the glycosaminoglycan mixture is obtained may be primary osteoblasts. The osteoblasts may be, or have been, maintained in *in vitro* cell culture. The cultured osteoblast cells from which the GAG mixture is obtained may be, or have been, in confluent or non-confluent cell culture.

In embodiments the osteoblast derived GAG mixture is a soluble osteoblast derived GAG mixture, e.g. obtained by the separation of a GAG fraction from culture media (preferably liquid, fluid or gel culture media) in which the osteoblasts are, or have been, cultured to form a soluble osteoblast derived GAG mixture.

The soluble osteoblast derived GAG mixture may have two peaks in Sepharose CL-6B size-exclusion column chromatography,

The soluble osteoblast derived GAG mixture may exhibit dose dependent binding to RANKL.

The soluble osteoblast derived GAG mixture may inhibit RANKL induced osteoclast formation.

The soluble osteoblast derived GAG mixture may function to oppose the inhibitory effect of RANKL on ERK activity.

The soluble osteoblast derived GAG mixture may dose-dependently increase proliferation of osteoblasts, e.g. in *in vitro* culture. For example, the soluble osteoblast derived GAG mixture may enhance osteoblast proliferation, *in vitro,* by at least 1.2 fold, more preferably one of at least 1.4, 1.6, 1.8 or 2.0 fold, compared to proliferation in the absence of the GAG mixture.

The soluble osteoblast derived GAG mixture may reduce induction of osteoclasts by RANKL by one of at least 10%, 20%, 30%, 40%, 50% or 60%, and up to 70%, compared with induction of osteoclasts by 5-day treatment of RAW264.7 cells in *in vitro* culture with 10ng/ml RANKL in the absence of an added GAG mixture.

Another osteoblast derived GAG mixture may be a cell-surface osteoblast derived GAG mixture, e.g. obtained by the separation of a GAG fraction that is attached to cultured osteoblast cells.

The cell-surface osteoblast derived GAG mixture may have only a single peak in Sepharose CL-6B size-exclusion column chromatography.

The cell-surface osteoblast derived GAG mixture may exhibit dose dependent binding to RANKL.

Wherein the osteoblast derived GAG mixture may inhibit RANKL induced osteoclast formation.

Wherein the osteoblast derived GAG mixture may function to oppose the inhibitory effect of RANKL on ERK activity.

The cell surface osteoblast derived GAG mixture may dose-dependently increase proliferation of osteoblasts, e.g. in *in vitro* culture. For example, the soluble osteoblast derived GAG mixture may enhance osteoblast proliferation, *in vitro,* by at least 1.2 fold, more preferably one of at least 1.4, 1.6, 1.8 or 2.0 fold, compared to proliferation in the absence of the GAG mixture.

The cell surface osteoblast derived GAG mixture may reduce induction of osteoclasts by RANKL by one of at least 10%, 20%, 30%, 40%, and up to 45%, compared with induction of osteoclasts by 5-day treatment of RAW264.7 cells in *in vitro* culture with 10ng/ml RANKL in the absence of an added GAG mixture.

The osteoblast derived GAG mixtures of the present disclosure preferably contain a mixture of at least two of a heparan sulfate, a chondroitin sulfate, a dermatan sulfate, a keratin sulfate and a hyaluronic acid. The GAG mixtures may contain only one of each of these sugar species (e.g. only one heparan sulfate and/or only one chondroitin sulfate and/or only one dermatan sulfate and/or only one keratin sulfate and/or only one hyaluronic acid) but may alternatively contain more than one type of one of these sugar species (e.g. more than one type of heparan sulfate and/or more than one type of chondroitin sulfate and/or more than one type of dermatan sulfate and/or more than one type of keratin sulfate and/or more than one type of hyaluronic acid) and may also contain other sugar or glycan species. Preferably, the GAG mixtures do not contain heparin (beyond trace amounts).

In further aspects of the present invention a soluble glycosaminglycan mixture is provided for use in the inhibition of osteoclastogenesis, and/or the enhancement of proliferation of osteoblasts, and/or the treatment or prevention of bone fracture or bone deterioration.

Accordingly, in one aspect a soluble glycosaminoglycan mixture obtained from mammalian osteoblasts for use in the inhibition of osteoclastogenesis in a mammal is provided.

In another aspect a soluble glycosaminoglycan mixture obtained from mammalian osteoblasts for use in the treatment and/or prevention of bone fracture or bone deterioration in a mammal is provided.

In another aspect a soluble glycosaminoglycan mixture obtained from mammalian osteoblasts for use in enhancing proliferation of osteoblasts in a mammal is provided.

In another aspect the use of a soluble glycosaminoglycan mixture obtained from mammalian osteoblasts in the manufacture of a medicament for the inhibition of osteoclastogenesis in a mammal is provided.

In another aspect the use of a soluble glycosaminoglycan mixture obtained from mammalian osteoblasts in the manufacture of a medicament for the treatment and/or prevention of bone fracture or bone deterioration in a mammal is provided.

In another aspect the use of a soluble glycosaminoglycan mixture obtained from mammalian osteoblasts in the manufacture of a medicament for enhancing proliferation of osteoblasts in a mammal is provided.

The methods and uses may comprise administering the glycosaminoglycan mixture(s) to bone tissue of the mammal or to tissue surrounding bone tissue of the mammal.

In another aspect a method of inhibiting osteoclastogenesis is provided, the method comprising administering a soluble glycosaminoglycan mixture obtained from mammalian osteoblasts to osteoclasts or osteoblasts in vitro. The method may be performed in vitro on cultured cells.

In another aspect a method of enhancing proliferation of osteoblasts is provided, the method comprising administering a soluble glycosaminoglycan mixture obtained from mammalian osteoblasts to osteoblasts in vitro. The method may be performed in vitro on cultured cells.

In a further aspect of the present disclosure a pharmaceutical composition or medicament is provided, the pharmaceutical composition or medicament comprising a glycosaminoglycan mixture according to any one of the aspects of the present invention described above.

The pharmaceutical composition or medicament may be provided for use in the inhibition of osteoclastogenesis in mammal, and/or in the treatment and/or prevention of bone fracture or bone deterioration in a mammal, and/or in enhancing proliferation of osteoblasts in a mammal.

The pharmaceutical compositions or medicaments may comprise the osteoblast derived GAG mixture together with one or more pharmaceutically acceptable carriers, adjuvants or diluents.

### Description of Preferred Embodiments

Skeletal integrity is tightly regulated by the activity of osteoblasts and osteoclasts that are both under the control of extracellular glycosaminoglycans (GAGs) through their interaction with endogenous growth factors. RANKL, a growth factor critical to osteoclast formation, is produced by osteoblasts and further modulated by certain types of GAGs. Instead of exploring how a single species of GAG can affect osteoclast formation, we isolated a pool of osteoblast-derived GAGs that we believe more fully represents the tissue microenvironment within which osteoclasts reside, and demonstrated that these GAGs block the osteoclastogenic activity of RANKL. Furthermore, we observed that RANKL significantly reduced ERK activity, a putative osteoclastogenesis suppressor; whereas osteoblast-derived GAG removed the inhibitory effect of RANKL on ERK activity. Notably, while imposing an anti-osteoclastic affect, these GAGs also enhanced the proliferation of osteoblasts. Therefore, the osteoblast microenvironment appears to contain a source of GAG favoring the anabolic activity of bone. Moreover, the anti-osteoclastogenic activity of these GAGs may provide a useful therapeutic tool for the treatment of osteopenia/ osteoporosis.

We extracted GAGs from the cell surface of osteoblasts as well as those secreted into the media in soluble forms. Their binding affinity to RANKL and the effect on RANKL induced osteoclast differentiation was then examined. To reduce potential contaminating effects from endogenous RANKL activity, we used osteoclastic precursor cells (RAW264.7) that lack RANKL or OPG expression but remain responsive to exogenous RANKL stimulation and possess osteoclastogenic potential [Fuller et al., 1998; Hsu et al., 1999; Yasuda et al., 1998]. Our study demonstrates that GAGs isolated from osteoblasts have affinity for RANKL and significantly inhibit RANKL-induced osteoclastogenesis by modulating ERK activity. These findings suggest that osteoblasts produce extracellular macromolecules to fine-tune osteoclast activity. Moreover the anti-osteoclastic activity of these osteoblast-derived GAGs supports their further development as substances for treating bone disorders with aberrantly elevated RANKL / osteoclast activity.

The glycosaminoglycan mixtures of the present invention provide a source of glycosaminoglycans for the treatment of diseases of bone loss. Bisphosphonates are typically used that are a class of drugs that prevent the loss of bone mass, and are used to treat osteoporosis and similar diseases.

Typically bone has a constant turnover, and is kept in balance (homeostasis) by osteoblasts creating bone and osteoclasts digesting bone. Bisphosphonates inhibit the digestion of bone by osteoclasts. Osteoclasts also have constant turnover and normally destroy themselves by a process called cell suicide (apoptosis). Bisphosphonates encourage osteoclasts to undergo apoptosis. We have found a source of GAG that, like Bisphosphonate, inhibits osteoclast development, but importantly, does not inhibit osteoblasts, thus the result is improved skeletal mass.

The uses of bisphosphonates include the prevention and treatment of osteoporosis, osteitis deformans ("Paget's disease of bone"), bone metastasis (with or without hypercalcaemia), multiple myeloma, primary hyperparathyroidism, osteogenesis imperfecta and other conditions that feature bone fragility.

### Osteoblast derived GAG mixtures

The present invention relates to glycosaminoglycan mixtures obtained from or obtainable from osteoblasts, and particularly to the use of such osteoblast derived GAG mixtures to inhibit osteoclastogenesis, and/or enhance proliferation of osteoblasts, and/or treat or prevent bone fracture or bone deterioration. The osteoblast derived GAG mixtures may be provided in isolated or substantially purified form.

A substantially purified form of an osteoblast derived GAG mixture may comprise a composition in which at least 80% of the glycosaminoglycan component is comprised of an osteoblast derived glycosaminoglycan mixture according to the present invention. More preferably, this will be one of at least 85%, 90%, 95%, 96%, 97%, 98%, 99% or 100%.

The osteoblast derived GAG mixture may be provided in substantially isolated form or as part of a composition comprising other components, e.g. a pharmaceutically acceptable carrier adjuvant or diluent.

The osteoblast derived GAG mixtures are preferably obtained from mammalian osteoblasts, more preferably from osteoblasts from a human or a non-human mammal (e.g. rabbit, guinea pig, rat, mouse or other rodent (including any animal in the order Rodentia), cat, dog, pig, sheep, goat, cattle (including cows, e.g. dairy cows, or any animal in the order *Bos*), horse (including any animal in the order *Equidae*), donkey, and non-human primate).

The osteoblasts may be primary osteoblasts and may be maintained in *in vitro* cell culture The osteoblast cells may be cultured in either confluent or non-confluent cell culture.

Osteoblast derived GAG mixtures may be obtained in one of three ways:
1. Separation of the GAG fraction from culture media in which the osteoblasts are cultured to form a "soluble osteoblast derived GAG mixture".
2. Separation of the GAG fraction attached to cultured osteoblast cells cultured to form a "cell surface osteoblast derived GAG mixture".
3. Separation of the extracellular matrix GAG fraction from cultured osteoblast cells to form an "extracellular matrix osteoblast derived GAG mixture".

A soluble osteoblast derived GAG mixture may be obtained by removing culture media from cultured osteoblast cells, then removing cell and other debris from the media and subjecting the remaining GAG containing fraction to anion exchange chromatography (e.g. on a CaptoQ-Sepaharose column equilibrated in 50mM PBS with 250 mM NaCl (low-salt buffer)). Glycosaminoglycans may optionally be released from proteoglycans in the eluate by enzymatic treatment (e.g. by treatment with neuraminidase (0.1 U) for 4 h followed by further digestion with 10 mg/ml Pronase (in 500 mM Tris-acetate, 50 mM calcium acetate, pH 8.0) and 5 volumes of 100 mM Tris-acetate (pH 8.0) at 37 °C for 24 h) optionally followed by further separation of the released glycosaminoglycans (e.g. by dilution 1:10 with low-salt buffer, and chromatographic separation e.g. by passing through a CaptoQ-Sepharose chromatography column (50 ml) and elution of glycosaminoglycans - as described above).

A cell surface osteoblast derived GAG mixture may be obtained by forming a cell solution of cultured osteoblasts, lysing the cells, collecting the lysate and extracting the GAG fraction by anion exchange chromatography (e.g. on a CaptoQ-Sepaharose column equilibrated in 50mM PBS with 250 mM NaCl (low-salt buffer)). Glycosaminoglycans may optionally be released from proteoglycans in the eluate by enzymatic treatment (e.g. by treatment with neuraminidase (0.1 U) for 4 h followed by further digestion with 10 mg/ml Pronase (in 500 mM Tris-acetate, 50 mM calcium acetate, pH 8.0) and 5 volumes of 100 mM Tris-acetate (pH 8.0) at 37 °C for 24 h) optionally followed by further separation of the released glycosaminoglycans (e.g. by dilution 1:10 with low-salt buffer, and chromatographic separation e.g. by passing through a CaptoQ-Sepharose chromatography column (50 ml) and elution of glycosaminoglycans - as described above).

The osteoblast derived GAG mixtures of the present disclosure can be characterized with reference to their structural properties. For example, a soluble osteoblast derived GAG mixture may have two peaks in Sepharose CL-6B size-exclusion column chromatography, with a cell surface osteoblast derived GAG mixture having only a single peak.

The osteoblast derived GAG mixtures of the present disclosure can also be characterized with reference to their functional properties.

For example, the soluble and/or cell surface osteoblast derived GAG mixtures may (preferably separately and individually) exhibit dose dependent binding to RANKL (10 to 150 ng/ml) when 5µg of the GAG mixture is pre-bound to a binding plate. Binding to RANKL may be direct and/or specific and/or competitive.

The soluble and/or cell surface osteoblast derived GAG mixtures may also (preferably separately and individually) inhibit RANKL induced osteoclast formation (e.g. at a concentration of 5µg/ml), as measured by *in vitro* differentiation of RAW264.7 cells into TRAP-positive multinucleated osteoclasts.

The soluble osteoblast derived GAG mixtures may reduce induction of osteoclasts by RANKL by up to 70%, and preferably by at least one of 10%, 20%, 30%, 40%, 50% or 60%, compared with induction of osteoclasts by 5-day treatment of RAW264.7 cells with 10ng/ml RANKL in the absence of an added GAG mixture.

The cell surface osteoblast derived GAG mixtures may reduce induction of osteoclasts by RANKL by up to 45%, and preferably by at least one of 10%, 20%, 30%, 40%, 50% or 60%, compared with induction of osteoclasts by 5-day treatment of RAW264.7 cells with 10ng/ml RANKL in the absence of an added GAG mixture.

The soluble and/or cell surface osteoblast derived GAG mixtures may also (preferably separately and individually) oppose the inhibitory effect of RANKL on ERK activity.

The soluble and/or cell surface osteoblast derived GAG mixtures may also (preferably separately and individually) dose-dependently increase proliferation of osteoblasts, e.g. in *in vitro* culture. For example, soluble and/or cell surface osteoblast derived GAG mixtures each (and separately) are preferably able to enhance osteoblast proliferation, *in vitro,* by at least 1.2 fold, more preferably one of at least 1.4, 1.6, 1.8 or 2.0 fold, compared to proliferation in the absence of the GAG mixture.

### Proteoglycans

Proteoglycans generally represent a special class of glycoproteins that are heavily glycosylated. They consist of a core protein with one or more covalently attached glycosaminoglycan (GAG) chain(s). These glycosaminoglycan chains are long, linear carbohydrate polymers that are negatively charged under physiological conditions, due to the occurrence of sulfate and uronic acid groups. Proteoglycans are a major component of the animal extracellular matrix. Therein, proteoglycans form large complexes, both to other proteoglycans and also to fibrous matrix proteins (such as collagen). They are also involved in binding cations (such as sodium, potassium and calcium) and water, and also regulate the movement of molecules through the matrix. Evidence also shows they can affect the activity and stability of proteins and signaling molecules within the matrix. Individual functions of proteoglycans can be attributed to either the protein core or the attached GAG chain.

Growth factors binding to proteoglycans of the extracellular matrix have been shown to regulate both differentiation and proliferation of human stem cells in vitro and in vivo. These growth factors signal through interaction with specific plasma membrane receptor kinases, an interaction that may involve proteoglycan binding. However, existing methods of using growth factors, such as FGF, have the drawback that while stimulating stem cell proliferation, the growth factor addition leads to a significant loss of multipotentiality of the stem cells. In contrast and surprisingly, the observed proliferation increase achieved according to the methods of the present invention is accompanied by the preservation of the multipotentiality of the stem cells.

Proteoglycans can be used in accordance with the present invention, even though they may in some preferred embodiments not be as conveniently used as the corresponding glycosaminoglycans, because the saccharide chains have been found to be easier to handle, i.e. smaller and more stable, more soluble and less prone to interfering interactions, for example with the extracellular matrix. Therefore, the glycosaminoglycans may have an increased bioactivity per microgram compared to the proteoglycans.

### Glycosaminglycans

As used herein, the terms 'glycosaminoglycan' and 'GAG' are used interchangeably and are understood to refer to the large collection of molecules comprising an oligosaccharide, wherein one or more of those conjoined saccharides possess an amino substituent, or a derivative thereof. Examples of GAGs are chondroitin sulfate, keratan sulfate, heparin, dermatan sulfate, hyaluronate and heparan sulfate.

GAG mixtures of the present invention are expected to contain a mixture of at least two of a heparan sulfate, a chondroitin sulfate, a dermatan sulfate, a keratin sulfate and a hyaluronic acid. The GAG mixtures may contain more than one type of one of these sugar species and may also contain other sugar or glycan species. Preferably, the GAG mixtures do not contain heparin (beyond trace amounts).

As used herein, the term 'GAG' also extends to encompass those molecules that are GAG conjugates. An example of a GAG conjugate is a proteoglycosaminoglycan (PGAG, proteoglycan) wherein a peptidic component is covalently bound to an oligosaccharide component.

In the present invention, it is understood that there are a large number of sources of GAG compounds including natural, synthetic or semi-synthetic. A preferred source of GAGs is biological tissue, particularly osteoblast cell(s), e.g. mammalian osteoblast cells e.g. human or non-human (e.g. rabbit, guinea pig, rat, mouse or other rodent (including any animal in the order Rodentia), cat, dog, pig, sheep, goat, cattle (including cows, e.g. dairy cows, or any animal in the order *Bos*), horse (including any animal in the order *Equidae*), donkey, and non-human primate) mammalian osteoblast cells.

GAGs may also be obtained from synthetic sources. In this respect, GAGs may be obtained from the synthetic elaboration of commercially available starting materials into more complicated chemical form through techniques known, or conceivable, to one skilled in the art. An example of such a commercially available starting material is glucosamine. Another preferred source of GAGs is a semi-synthetic source. In this respect, synthetic elaboration of a natural starting material, which possesses much of the complexity of the desired material, is elaborated synthetically using techniques known, or conceivable, to one skilled in the art. Examples of such a natural starting material are chitin and dextran, and examples of the types of synthetic steps that may elaborate that starting material, into a GAG mixture suitable for use in the present invention, are amide bond hydrolysis, oxidation and sulfation. Another example of a semi-synthetic route to GAGs of the desired structure comprises the synthetic interconversion of related GAGs to obtain GAGs suitable for use in the present invention.

Heparan sulphate is one type of glycosaminoglycan.

Heparan sulfate proteoglycans (HSPGs) represent a highly diverse subgroup of proteoglycans and are composed of heparan sulfate glycosaminoglycan side chains covalently attached to a protein backbone. The core protein exists in three major forms: a secreted form known as perlecan, a form anchored in the plasma membrane known as glypican, and a transmembrane form known as syndecan. They are ubiquitous constituents of mammalian cell surfaces and most extracellular matrices. There are other proteins such as agrin, or the amyloid precursor protein, in which an HS chain may be attached to less commonly found cores.

"Heparan Sulphate" ("Heparan sulfate" or "HS") is initially synthesised in the Golgi apparatus as polysaccharides consisting of tandem repeats of D-glucuronic acid (GlcA) and N-acetyl-D-glucosamine (GlcNAc). The nascent polysaccharides may be subsequently modified in a series of steps: N-deacetylation/N-sulfation of GlcNAc, C5 epimerisation of GlcA to iduronic acid (IdoA), O-sulphation at C2 of IdoA and GlcA, O-sulphation at C6 of N-sulphoglucosamine (GlcNS) and occasional O-sulphation at C3 of GlcNS. N-deacetylation/N-sulphation, 2-O-, 6-O- and 3-O-sulphation of HS are mediated by the specific action of HS N-deacetylase/N-sulfotransferase (HSNDST), HS 2-O-sulfotransferase (HS2ST), HS 6-O-sulfotransferase (HS6ST) and HS 3-O-sulfotransferase, respectively. At each of the modification steps, only a fraction of the potential substrates are modified, resulting in considerable sequence diversity. This structural complexity of HS has made it difficult to determine its sequence and to understand the relationship between HS structure and function.

Heparan sulfate side chains consist of alternately arranged D-glucuronic acid or L-iduronic acid and D-glucosamine, linked via (1 -> 4) glycosidic bonds. The glucosamine is often N-acetylated or N-sulfated and both the uronic acid and the glucosamine may be additionally O-sulfated. The specificity of a particular HSPG for a particular binding partner is created by the specific pattern of carboxyl, acetyl and sulfate groups attached to the glucosamine and the uronic acid. In contrast to heparin, heparan sulfate contains less N- and O-sulfate groups and more N-acetyl groups. The heparan sulfate side chains are linked to a serine residue of the core protein through a tetrasaccharide linkage (-glucuronosyl-β-(1→ 3)-galactosyl-β-(1→ 3)-galactosyl-β-(1→ 4)-xylosyl-β-1-O-(Serine)) region.

Both heparan sulfate chains and core protein may undergo a series of modifications that may ultimately influence their biological activity. Complexity of HS has been considered to surpass that of nucleic acids (Lindahl et al, 1998, J. Biol. Chem. 273, 24979; Sugahara and Kitagawa, 2000, Curr. Opin. Struct. Biol. 10, 518). Variation in HS species arises from the synthesis of non-random, highly sulfated sequences of sugar residues which are separated by unsulfated regions of disaccharides containing N- acetylated glucosamine. The initial conversion of N-acetylglucosamine to N- sulfoglucosamine creates a focus for other modifications, including epimerization of glucuronic acid to iduronic acid and a complex pattern of O-sulfations on glucosamine or iduronic acids. In addition, within the non-modified, low sulfated, N-acetylated sequences, the hexuronate residues remain as glucuronate, whereas in the highly sulfated N-sulfated regions, the C-5 epimer iduronate predominates. This limits the number of potential disaccharide variants possible in any given chain but not the abundance of each. Most modifications occur in the N-sulfated domains, or directly adjacent to them, so that in the mature chain there are regions of high sulfation separated by domains of low sulfation (Brickman et al. (1998), J. Biol. Chem. 273(8), 4350-4359).

It is hypothesized that the highly variable heparan sulfate chains play key roles in the modulation of the action of a large number of extracellular ligands, including regulation and presentation of growth and adhesion factors to the cell, via a complicated combination of autocrine, juxtacrine and paracrine feedback loops, so controlling intracellular signaling and thereby the differentiation of stem cells. For example, even though heparan sulfate glycosaminoglycans may be genetically described (Alberts et al. (1989) Garland Publishing, Inc, New York & London, pp. 804 and 805), heparan sulfate glycosaminoglycan species isolated from a single source may differ in biological activity. As shown in Brickman et al, 1998, Glycobiology 8, 463, two separate pools of heparan sulfate glycosaminoglycans obtained from neuroepithelial cells could specifically activate either FGF-1 or FGF-2, depending on mitogenic status. Similarly, the capability of a heparan sulfate (HS) to interact with either FGF-1 or FGF-2 is described in WO 96/23003. According to this patent application, a respective HS capable of interacting with FGF-1 is obtainable from murine cells at embryonic day from about 11 to about 13, whereas a HS capable of interacting with FGF-2 is obtainable at embryonic day from about 8 to about 10.

As stated above HS structure is highly complex and variable between HS. Indeed, the variation in HS structure is considered to play an important part in contributing toward the different activity of each HS in promoting cell growth and directing cell differentiation. The structural complexity is considered to surpass that of nucleic acids and although HS structure may be characterised as a sequence of repeating disaccharide units having specific and unique sulfation patterns at the present time no standard sequencing technique equivalent to those available for nucleic acid sequencing is available for determining HS sequence structure. In the absence of simple methods for determining a definitive HS sequence structure HS molecules are positively identified and structurally characterised by skilled workers in the field by a number of analytical techniques. These include one or a combination of disaccharide analysis, tetrasaccharide analysis, HPLC and molecular weight determination. These analytical techniques are well known to and used by those of skill in the art.

Two techniques for production of di- and tetra- saccharides from HS include nitrous acid digestion and lyase digestion. A description of one way of performing these digestion techniques is provided below, purely by way of example, such description not limiting the scope of the present invention.

### Nitrous acid digestion

Nitrous acid based depolymerisation of heparan sulphate leads to the eventual degradation of the carbohydrate chain into its individual disaccharide components when taken to completion.

For example, nitrous acid may be prepared by chilling 250 µl of 0.5 M H₂SO₄ and 0.5 M Ba(NO₂)₂ separately on ice for 15 min. After cooling, the Ba(NO₂)₂ is combined with the H₂SO₄ and vortexed before being centrifuged to remove the barium sulphate precipitate. 125 µl of HNO₂ was added to GAG samples resuspended in 20 µl of H₂O, and vortexed before being incubated for 15 min at 25°C with occasional mixing. After incubation, 1 M Na₂CO₃ was added to the sample to bring it to pH 6. Next, 100 µl of 0.25 M NaBH₄ in 0.1 M NaOH is added to the sample and the mixture heated to 50°C for 20 min. The mixture is then cooled to 25°C and acidified glacial acetic acid added to bring the sample to pH 3. The mixture is then neutralised with 10 M NaOH and the volume decreased by freeze drying. Final samples are run on a Bio-Gel P-2 column to separate di- and tetrasaccharides to verify the degree of degradation.

### Lyase digestion

Heparinise III cleaves sugar chains at glucuronidic linkages. The series of Heparinase enzymes (I, II and III) each display relatively specific activity by depolymerising certain heparan sulphate sequences at particular sulfation recognition sites. Heparinase I cleaves HS chains with NS regions along the HS chain. This leads to disruption of the sulphated domains. Heparinase III depolymerises HS with the NA domains, resulting in the separation of the carbohydrate chain into individual sulphated domains. Heparinase II primarily cleaves in the NA/NS "shoulder" domains of HS chains, where varying sulfation patterns are found. Note: The repeating disaccharide backbone of the heparan polymer is a uronic acid connected to the amino sugar glucosamine. "NS" means the amino sugar is carrying a sulfate on the amino group enabling sulfation of other groups at C2, C6 and C3. "NA" indicates that the amino group is not sulphated and remains acetylated.

For example, for depolymerisation in the NA regions using Heparinase III both enzyme and lyophilised HS samples are prepared in a buffer containing 20 mM Tris-HCL, 0.1 mg/ml BSA and 4 mM CaCl₂ at pH 7.5. Purely by way of example, Heparinase III may be added at 5 mU per 1µg of HS and incubated at 37°C for 16 h before stopping the reaction by heating to 70°C for 5 min.

Di- and tetrasaccharides may be eluted by column chromatography.

### Medical Use

The osteoblast derived GAG mixtures of the present disclosure are each (separately, but optionally in combination) provided for use in one or more of the inhibition of osteoclastogenesis, the enhancement of proliferation of osteoblasts, and/or the treatment or prevention of bone fracture or bone deterioration. As such the osteoblast derived GAG mixtures of the present disclosure may be provided for use in a method of medical treatment. Treatment may involve administration of the osteoblast derived GAG mixture, or a preparation or composition comprising such to a patient in need of treatment.

### Prevention of Osteoclastogenesis

The osteoblast derived GAG mixtures of the present disclosure (soluble and/or cell surface) may be used in reducing or inhibiting the processes of osteoclastogenesis and/or bone resoprtion.

Osteoclastogenesis is the process through which osteoclasts are formed, usually through the interaction of bone marrow stromal cells with haematopoietic osteoclast precursors.

The inventors have shown that the soluble and cell surface osteoblast derived GAG mixtures are each (separately) capable of reducing the process of osteoclastogenesis, thereby having an anti-osteoporotic effect.

### Promotion of Bone formation (Osteoblastogenesis)

The inventors have also shown that the soluble and cell surface osteoblast derived GAG mixtures are each (separately) capable of promoting proliferation of osteoblasts, which is necessary for bone formation. They have each (separately) been shown to shift the balance of bone remodeling towards bone formation by favoring osteoblastogenesis while antagonizing osteoclastogenesis.

### Bone Fracture and bone deterioration

Accordingly, in some aspects the present disclosure is concerned with the therapeutic use (human and/or veterinary) of the osteoblast derived GAG mixtures of the present invention (soluble and/or cell surface osteoblast derived GAG mixtures, each separately and individually, although optionally in combination) to treat or prevent bone fracture and/or bone deterioration.

The osteoblast derived GAG mixtures each stimulate osteoblast proliferation and inhibit osteoclastogenesis.

Bone fracture is a medical condition. In this application "fracture" includes damage or injury to bone in which a bone is cracked, broken or chipped. A break refers to discontinuity in the bone. A fracture may be caused by physical impact, or mechanical stress or by medical conditions such as osteoporosis or osteoarthritis.

Orthopaedic classification of fractures includes closed or open and simple or multi-fragmentary fractures. In closed fractures the skin remains intact, whilst in an open fracture the bone may be exposed through the wound site, which brings a higher risk of infection. Simple fractures occur along a single line, tending to divide the bone in two. Multi-fragmentary fractures spilt the bone into multiple pieces.

Other fracture types include, compression fracture, compacted fracture, spiral fracture, complete and incomplete fractures, transverse, linear and oblique fractures and comminuted fractures.

In most subjects bone healing (fracture union) occurs naturally and is initiated following injury. Bleeding normally leads to clotting and attraction of white blood cells and fibroblasts, followed by production of collagen fibres. This is followed by bone matrix (calcium hydroxyapatite) deposition (mineralisation) transforming the collagen matrix into bone. Immature re-generated bone is typically weaker than mature bone and over time the immature bone undergoes a process of remodelling to produce mature "lamellar" bone. The complete bone healing process takes considerable time, typically many months.

Bones in which fractures or deterioration occurs and which may benefit from treatment using the osteoblast derived GAG mixtures of the present invention include all bone types, particularly all mammalian bones including, but not limited to, long bones (e.g. femur, humerus, phalanges), short bones (e.g. carpals, tarsals), flat bones (e.g. cranium, ribs, scapula, sternum, pelvic girdle), irregular bones (e.g. vertebrae), sesamoid bones (e.g. patella).

Bones in which fractures or deterioration occurs and which may benefit from treatment using the osteoblast derived GAG mixtures of the present invention include skeletal bone (i.e. any bone of the skeleton), bones of the cranio-facial region, bones of the axial skeleton (e.g. vertebrae, ribs), appendicular bone (e.g. of the limbs), bone of the pelvic skeleton (e.g. pelvis).

Bones in which fractures or deterioration occurs and which may benefit from treatment using the osteoblast derived GAG mixtures of the present invention also include those of the head (skull) and neck, including those of the face such as the jaw, nose and cheek.

Bone fracture or bone deterioration also includes pathological porosity, such as that exhibited by subjects with osteoporosis or other forms of bone resorption.

The osteoblast derived GAG mixtures (soluble and/or cell surface osteoblast derived GAG mixtures, each separately and individually, although optionally in combination) are also provided for the prevention and/or treatment of one or more of: osteoporosis, lytic bone metastases, rheumatoid arthritis, postmenopausal osteoporosis, glucocorticoid-induced osteoporosis, multiple myeloma associated osteolytic lesions, atherosclerotic disease associated vascular calcification, osteopenia, bone disorders with aberrantly elevated RANKL / osteoclast activity, or bone deterioration associated with any of these conditions.

The osteoblast derived GAG mixtures (soluble and/or cell surface osteoblast derived GAG mixtures, each separately and individually, although optionally in combination) are also provided for the prevention and/or treatment of osteoporosis, osteitis deformans ("Paget's disease of bone"), bone metastasis (with or without hypercalcaemia), multiple myeloma, primary hyperparathyroidism, osteogenesis imperfecta and other conditions that feature bone fragility.

Although not limiting to the present invention, the primary actions of the osteoblast derived GAG mixtures of the present invention may be on cells within, adjacent to, or caused to migrate into a wound site and may be on the bone stem cells, the preosteoblasts, the osteoblasts, the osteoclasts, on any of the ancillary or vasculogenic cells found or caused to migrate into or within the wound bed, on RANK, RANKL or OPG.

Each of the osteoblast derived GAG mixtures of the present invention and pharmaceutical compositions and medicaments comprising one or more of the osteoblast derived GAG mixtures of the present invention are provided for use in a method of treatment of bone fracture or bone deterioration in a mammalian subject.

Treatment may comprise wound healing in bone. The treatment may involve repair, regeneration and growth of bone. The treatment may involve inhibition of bone resorption.

Treatment may also include treatment and/or prevention of osteoporosis or osteoarthritis.

Administration of the osteoblast derived GAG mixture is preferably to the tissue surrounding the fracture. This may include administration directly to bone tissue in which the fracture or deterioration has occurred. Administration may be to connective tissue surrounding the bone or fracture or to vasculature (e.g. blood vessels) near to and supplying the bone. Administration may be directly to the site of injury and may be to a callus formed by initial healing of the wound.

Medicaments and pharmaceutical compositions according to the present invention may be formulated for administration by a number of routes. Most preferably the osteoblast derived GAG mixture is formulated in fluid or liquid form for injection.

In some embodiments the osteoblast derived GAG mixture is formulated as a controlled release formulation, e.g. in a drug capsule for implantation at the wound site. The osteoblast derived GAG mixture may be attached to, impregnated on or soaked into a carrier material (e.g. a biomaterial) such as nanofibres or biodegradable paper or textile.

Administration is preferably in a "therapeutically effective amount", this being sufficient to improve healing of the bone fracture compared to a corresponding untreated fracture. The actual amount administered, and rate and time-course of administration, will depend on the nature and severity of the fracture. Prescription of treatment, e.g. decisions on dosage etc, is within the responsibility of general practitioners and other medical doctors, and will typically take account of the nature of the fracture, the condition of the individual patient, the site of delivery, the method of administration and other factors known to practitioners. Single or multiple administrations of doses of the osteoblast derived GAG mixture may be administered in accordance with the guidance of the prescribing medical practitioner. Purely by way of example, the osteoblast derived GAG mixture may be delivered in dosages of at least 1 ng/ml, more preferably at least 5ng/ml and optionally 10 ng/ml or more. Individual dosages may be of the order less than 1 mg and greater than 1µg, e.g. one of about 5µg, about 10µg, about 25µg, about 30µg, about 50µg, about 100µg, about 0.5mg, or about 1 mg. Examples of the techniques and protocols mentioned above can be found in Remington's Pharmaceutical Sciences, 20th Edition, 2000, pub. Lippincott, Williams & Wilkins.

The osteoblast derived GAG mixture may be used to treat bone fracture or bone deterioration alongside other treatments, such as administration of pain relieving or antiinflammatory medicaments, immobilisation and setting of the bone, e.g. immobilising the injured limb in a plaster cast, surgical intervention, e.g. to re-set a bone or move a bone to correct displacement, angulation or dislocation. If surgery is required the osteoblast derived GAG mixture may be administered directly to (e.g. applied to) the fracture during the surgical procedure.

### Biomaterials

Pharmaceutical compositions and medicaments may take the form of a biomaterial that is coated and/or impregnated with an osteoblast derived GAG mixture. An implant or prosthesis may be formed from the biomaterial. Such implants or prostheses may be surgically implanted to assist in bone growth, regeneration, restructuring and/or remodelling.

The osteoblast derived GAG mixture may be applied to implants or prostheses to accelerate new bone formation and/or prevent further bone deterioration at a desired location. It will be appreciated that glycosaminpglycans, unlike proteins, are particularly robust and have a much better ability to withstand the solvents required for the manufacture of synthetic bioscaffolds and application to implants and prostheses.

The biomaterial may be coated or impregnated with the osteoblast derived GAG mixture. Impregnation may comprise forming the biomaterial by mixing the osteoblast derived GAG mixture with the constitutive components of the biomaterial, e.g. during polymerisation, or absorbing the osteoblast derived GAG mixture into the biomaterial. Coating may comprise adsorbing the osteoblast derived GAG mixture onto the surface of the biomaterial.

The biomaterial should allow the coated or impregnated osteoblast derived GAG mixture to be released from the biomaterial when administered to or implanted in the subject. Biomaterial release kinetics may be altered by altering the structure, e.g. porosity, of the biomaterial.

In addition to coating or impregnating a biomaterial with the osteoblast derived GAG mixture, one or more biologically active molecules may be impregnated or coated on the biomaterial. For example, at least one chosen from the group consisting of: BMP-2, BMP-4, OP-1, FGF-1, FGF-2, TGF-β1, TGF-β2, TGF-β3; VEGF; collagen; laminin; fibronectin; vitronectin. In addition or alternatively to the above bioactive molecules, one or more bisphosphonates may be impregnated or coated onto the biomaterial along with the osteoblast derived GAG mixture. Examples of useful bisphosphonates may include at least one chosen from the group consisting of: etidronate; clodronate; alendronate; pamidronate; risedronate; zoledronate.

Biomaterials coated or impregnated with the osteoblast derived GAG mixture may be useful in both medical and veterinary purposes. It will be appreciated that the present invention may improve the quality of life of a patient or potentially extend the life of an animal, for example a valuable racehorse for use in breeding.

The biomaterial provides a scaffold or matrix support. The biomaterial may be suitable for implantation in tissue, or may be suitable for administration (e.g. as microcapsules in solution).

The implant or prosthesis should be biocompatible, e.g. non-toxic and of low immunogenicity (most preferably non-immunogenic). The biomaterial may be biodegradable such that the biomaterial degrades as wound healing occurs, ultimately leaving only the regenerated bone *in situ* in the subject. Alternatively a non-biodegradable biomaterial may be used, e.g. to guide bone regeneration over a large discontinuity and/or to act as a structural support during bone healing, with surgical removal of the biomaterial being an optional requirement after successful wound healing.

Biomaterials may be soft and/or flexible, e.g. hydrogels, fibrin web or mesh, or collagen sponges. A "hydrogel" is a substance formed when an organic polymer, which can be natural or synthetic, is set or solidified to create a three-dimensional open-lattice structure that entraps molecules of water or other solutions to form a gel. Solidification can occur by aggregation, coagulation, hydrophobic interactions or cross-linking.

Alternatively biomaterials may be relatively rigid structures, e.g. formed from solid materials such as plastics or biologically inert metals such as titanium.

The biomaterial may have a porous matrix structure which may be provided by a crosslinked polymer. The matrix is preferably permeable to nutrients and growth factors required for bone growth.

Matrix structures may be formed by crosslinking fibres, e.g. fibrin or collagen, or of liquid films of sodium alginate, chitosan, or other polysaccharides with suitable crosslinkers, e.g. calcium salts, polyacrylic acid, heparin. Alternatively scaffolds may be formed as a gel, fabricated by collagen or alginates, crosslinked using well established methods known to those skilled in the art.

Suitable polymer materials for matrix formation include, but are not limited by, biodegradable/bioresorbable polymers which may be chosen from the group of: agarose, collagen, fibrin, chitosan, polycaprolactone, poly(DL-lactide-co-caprolactone), poly(L-lactide-co-caprolactone-co-glycolide), polyglycolide, polylactide, polyhydroxyalcanoates, co-polymers thereof, or non-biodegradable polymers which may be chosen from the group of: cellulose acetate; cellulose butyrate, alginate, polysulfone, polyurethane, polyacrylonitrile, sulfonated polysulfone, polyamide, polyacrylonitrile, polymethylmethacrylate, co-polymers thereof.

Collagen is a promising material for matrix construction owing to its biocompatibility and favourable property of supporting cell attachment and function (U.S. Pat. No. 5,019,087; Tanaka, S.; Takigawa, T.; Ichihara, S. & Nakamura, T. Mechanical properties of the bioabsorbable polyglycolic acid-collagen nerve guide tube Polymer Engineering & Science 2006, 46, 1461-1467). Clinically acceptable collagen sponges are one example of a matrix and are well known in the art (e.g. from Integra Life Sciences).

Fibrin scaffolds (e.g. fibrin glue) provide an alternative matrix material. Fibrin glue enjoys widespread clinical application as a wound sealant, a reservoir to deliver growth factors and as an aid in the placement and securing of biological implants (Rajesh Vasita, Dhirendra S Katti. Growth factor delivery systems for tissue engineering: a materials perspective. Expert Reviews in Medical Devices. 2006; 3(1): 29-47; Wong C, Inman E, Spaethe R, Helgerson S. Thromb.Haemost. 2003 89(3): 573-582; Pandit AS, Wilson DJ, Feldman DS. Fibrin scaffold as an effective vehicle for the delivery of acidic growth factor (FGF-1). J. Biomaterials Applications. 2000; 14(3); 229-242; DeBlois Cote MF. Doillon CJ. Heparin-fibroblast growth factor fibrin complex: in vitro and in vivo applications to collagen based materials. Biomaterials. 1994; 15(9): 665-672.).

Luong-Van et al (In vitro biocompatibility and bioactivity of microencapsulated heparan sulphate Biomaterials 28 (2007) 2127-2136) describes prolonged localised delivery of HS from polycaprolactone microcapsules.

A further example of a biomaterial is a polymer that incorporates hydroxyapatite or hyaluronic acid.

One example of a biomaterial suitable for use in combination with the osteoblast derived GAG mixture is the JAX™ bone void filler (Smith & Nephew). Jax granules are composed of high purity calcium sulfate and retain their shape to provide a scaffold with controlled, inter-granular porosity and granule migration stability. Jax granules dissolve safely and completely in the body.

Other suitable biomaterials include ceramic or metal (e.g. titanium), hydroxyapatite, tricalcium phosphate, demineralised bone matrix (DBM), autografts (i.e. grafts derived from the patient's tissue), or allografts (grafts derived from the tissue of an animal that is not the patient). Biomaterials may be synthetic (e.g. metal, fibrin, ceramic) or biological (e.g. carrier materials made from animal tissue, e.g. non-human mammals (e.g. cow, pig), or human).

The biomaterial can be supplemented with additional cells. For example, one can "seed" the biomaterial (or co-synthesise it) with undifferentiated bone precursor cells, e.g. stem cells such as mesenchymal stem cells, more preferably human mesenchymal stem cells.

The subject to be treated may be any animal or human. The subject is preferably mammalian, more preferably human. The subject may be a non-human mammal (e.g. rabbit, guinea pig, rat, mouse or other rodent (including cells from any animal in the order Rodentia), cat, dog, pig, sheep, goat, cattle (including cows, e.g. dairy cows, or any animal in the order *Bos*), horse (including any animal in the order *Equidae*), donkey, and non-human primate). The non-human mammal may be a domestic pet, or animal kept for commercial purposes, e.g. a race horse, or farming livestock such as pigs, sheep or cattle. The subject may be male or female. The subject may be a patient.

### Other uses

The osteoblast derived GAG mixtures are each (separately, but optionally in combination) provided for use in one or more of the inhibition of osteoclastogenesis, or the enhancement of proliferation of osteoblasts in *in vitro* culture or in other non-medical applications. Culture media may be provided for such purposes containing a predetermined quantity of one or more of the osteoblast derived GAG mixtures.

Methods and uses according to the present invention may be performed in vitro or in vivo. The term "in vitro" is intended to encompass experiments with cells in culture whereas the term "in vivo" is intended to encompass experiments with intact multi-cellular organisms.

### Culture Media

Culture media comprising the osteoblast derived GAG mixture may be of any kind but is preferably liquid or gel and may contain other nutrients and growth factors (e.g. FGF-2). The osteoblast derived GAG mixture will preferably be present in non-trace amounts. For example, the concentration of the osteoblast derived GAG mixture in the culture media may range between about 1.0 ng/ml culture media to about 1000 ng/ml culture media. Preferably, the concentration of the osteoblast derived GAG mixture in the culture media is between about 5 ng/ml culture media and 200 ng/ml culture media, more preferably between about 20 ng/ml culture media and 170 ng/ml culture media.

The invention includes the combination of the aspects and preferred features described except where such a combination is clearly impermissible or expressly avoided.

The section headings used herein are for organizational purposes only and are not to be construed as limiting the subject matter described.

Aspects and embodiments of the present invention will now be illustrated, by way of example, with reference to the accompanying figures. Further aspects and embodiments will be apparent to those skilled in the art.

### Brief Description of the Figures

Embodiments and experiments illustrating the principles of the invention will now be discussed with reference to the accompanying figures in which:

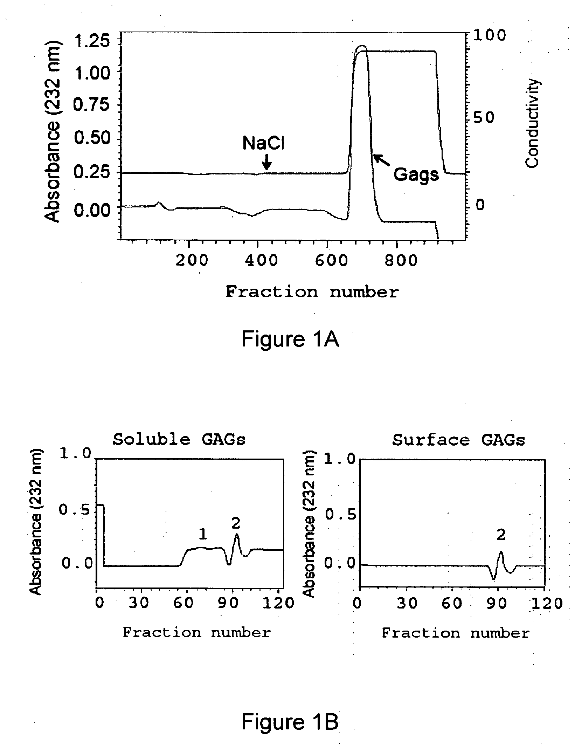

**Figure 1****.** Anion exchange chromatography of soluble and cell surface GAGs from primary osteoblasts. The crude GAGs were purified from porcine primary osteoblast culture media (soluble fraction) or cell extracts (cell surface fraction). **A,** Samples were applied to a 50 ml CaptoQ-Sepharose column at 4 ml/min in the presence of 1 M NaCl. During washing, a small amount of GAG eluted at absorbance of 232 nm and generated a peak from fraction number 640 to 760. **B,** Sepharose CL-6B size-exclusion column chromatogram of the GAGs isolated from soluble and cell surface fractions. Size fractions of soluble GAG chains displayed two peaks (1 and 2). Cell surface GAG chains displayed only one peak (2). The deflections below baseline represent monitor artifact.

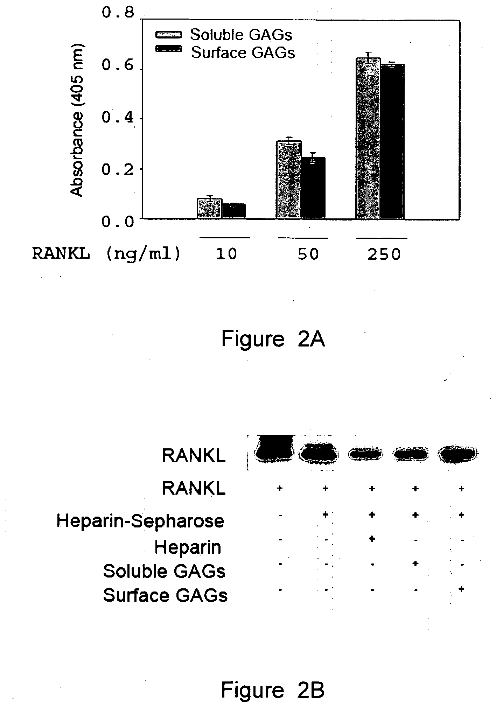

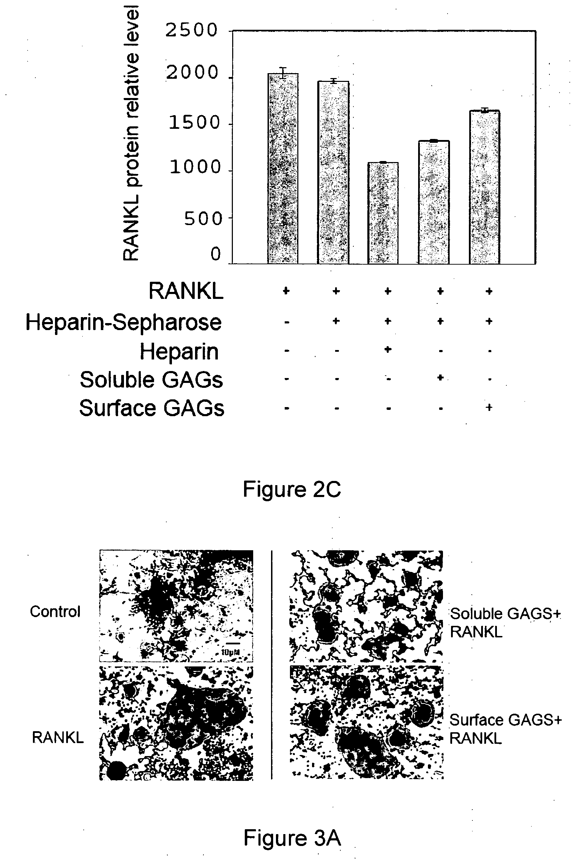

**Figure 2****.** Binding ability of GAGs to RANKL. **A,** 10 ng, 50 ng and 250 ng of RANKL was added to the plate coated with 5 µg soluble or cell surface GAG. The enzyme based assay was performed as described in "Materials and Methods" to detect the amount of RANKL bound to the immobilized GAG. Data represents the mean ± S.E. of triplicate experiments. **B** and **C,** 10 ng RANKL was added to heparin-Sepharose beads with or without 5 µg/ml free heparin or GAG. Lane 1 was RANKL protein as a positive control. RANKL bound to the beads was visualized by Western blot analysis. Data represents three independent experiments. Densitometry of RANKL binding represents the average of three independent experiments.

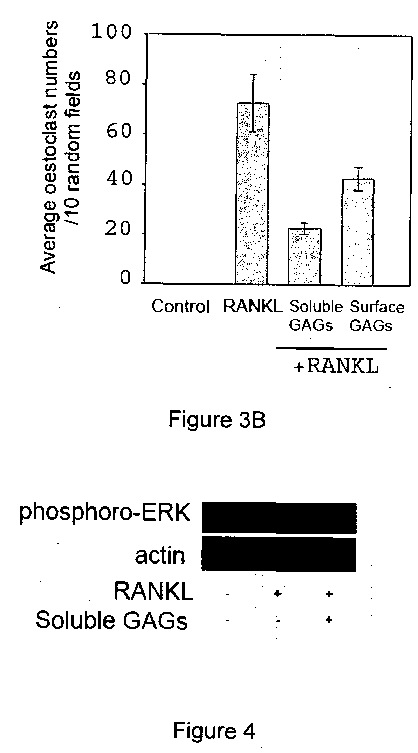

**Figure 3****.** The effect of GAGs on RANKL-induced osteoclast formation. RAW264.7 cells were cultured for 5 days in the presence of 10 ng/ml RANKL, or 10 ng/ml RANKL together with either 5 µg/ml soluble GAG or 5 µg/ml surface GAG. The cells without any treatment served as the control. Osteoclastogenesis was monitored by TRAP staining as described in "Materials and Methods". **A,** RAW264.7 monocytic cells were negative in TRAP staining (control). After 5 days of treatment with 10 ng/ml RANKL, numerous TRAP positive, multinucleated cells (seen in all cultures with RANKL) were observed. All images are from randomly chosen fields using a 10x objective. Data represents three independent experiments. **B,** GAGs from osteoblasts inhibited RANKL-induced osteoclastic differentation of RAW264.7 cells. Data represents the mean ± S.E. of triplicate experiments.
**Figure 4****.** The effect of soluble GAGs on RANKL-induced ERK activity. RAW264.7 cells were cultured for 5 days in the presence of 10 ng/ml RANKL, or 10 ng/ml RANKL together with 5 µg/ml soluble GAG. Western blot analysis demonstrated that RANKL antagonizes ERK phosphorylation, whereas the osteoblast-derived soluble GAG reduces the inhibitory effect of RANKL on ERK activity. Data represents three independent experiments.

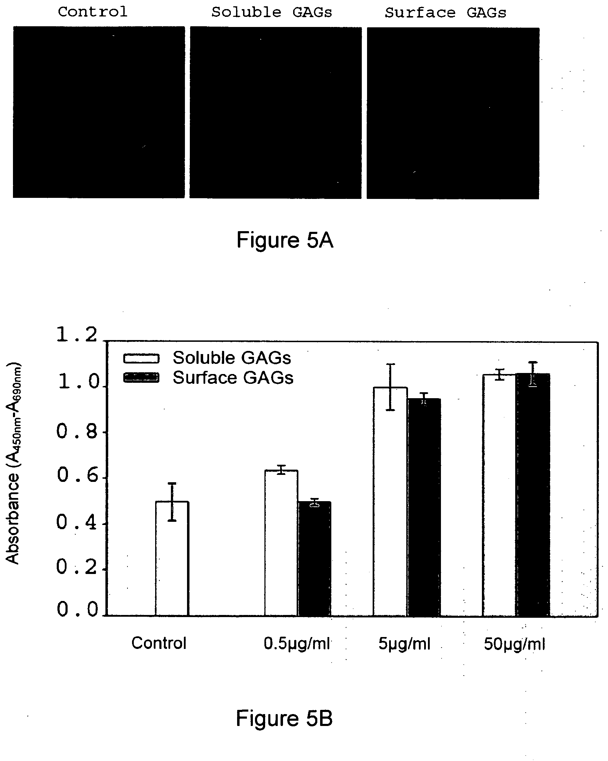

**Figure 5****.** The effect of GAGs on osteoblast proliferation. **A,** Primary human osteoblasts were growth for 2 days with or without 5 µg/ml soluble or cell surface GAG. Cells left untreated served as control. The actin cytoskeleton and the nuclei of the cells were stained with rhodamine phalloidin or DAPI, respectively and the images obtained by confocal microscopy. **B,** Primary human osteoblasts were deprived of serum to synchronize and arrest the cell growth for 24 hrs before being treated with 0.5, 5, 50 µg/ml soluble or cell surface GAGs. After 24 hrs, cell proliferation was determined by BrdU incorporation assay. The cells without any treatment served as the control. Data represents the mean ± S.E. of triplicate experiments.

### Detailed Description of the Invention

The details of one or more embodiments of the invention are set forth in the accompanying description below including specific details of the best mode contemplated by the inventors for carrying out the invention, by way of example. It will be apparent to one skilled in the art that the present invention may be practiced without limitation to these specific details.

### MATERIALS AND METHODS

### Extraction of GAGs from Porcine Osteoblasts

To obtain GAGs from primary osteoblasts, porcine primary osteoblast cultures was established as described previously [Manton et al., 2006] with strict adherence to the ethical guidelines of the Biomedical Research Council of Singapore and the National University of Singapore. Osteoblasts were collected from pre-confluent cultures and the expression of alkaline phosphatase used to confirm an osteoblastic phenotype. The osteoblasts obtained were then passaged and seeded at 5,000 cells per cm² and cultured in alpha MEM 10%FCS for 8 days (∼80 % confluent) with a media change every 3 days prior to GAG extraction. The media containing the soluble GAG fraction was gently removed from the culture dishes without disturbing the cells. To remove any cell debris, the media were centrifuged at maximum speed and the supernatants passed through a 0.45 µm filter. The cell monolayer was then washed with PBS and removed by trypsin-EDTA. The resulting cell solution was boiled for 10 min to deactivate the trypsin and the lysate collected by centrifugation at maximum speed for 15 min. The supernatant containing the cell surface GAG fraction was then passed through a 0.45 µm filter to remove any debris. Thereafter, the supernatant obtained from media (soluble fraction) or cells (cell surface fraction) was subjected to anionic-exchange chromatography on a CaptoQ-Sepharose column (50 ml) equilibrated in 50 mM PBS with 250 mM NaCl (low-salt buffer). Samples were loaded at a flow rate of 4 ml/min and the column washed with the same buffer until baseline was established. The bound material was eluted with 1 M NaCl (high-salt buffer) and the peak fractions pooled, concentrated, and desalted with distilled H₂O using HiPrep Desalting 16/10 Columns (GE Life-Sciences) as per manufacturer's instructions. The proteoglycan samples from the soluble and cell surface fractions were freeze-dried to be concentrated. The samples were then treated with neuraminidase (0.1 U) for 4 h followed by further digestion with 10 mg/ml Pronase (in 500 mM Tris-acetate, 50 mM calcium acetate, pH 8.0) and 5 volumes of 100 mM Tris-acetate (pH 8.0) at 37 °C for 24 h. The entire mixture was then diluted 1:10 with low-salt buffer, passed through a CaptoQ-Sepharose column (50 ml) and eluted as described previously. The peak fractions were pooled, concentrated, and desalted with water, freeze-dried and stored at -20 °C. The purified GAG chains from soluble and cell surface fractions were further characterized by size exclusion chromatography on a Sepharose CL-6B column (1 X 120 cm).

### Cell Culture

The RAW264.7 murine monocytic cell line (ATCC TIB-71, USA) was routinely cultured in Dulbecco's modified Eagle's media (DMEM) supplemented with 10 % fetal bovine serum (FBS) and 100 units/ml penicillin/streptomycin at 37 °C in a humidified 5% CO₂ incubator. For experiments, RAW 264.7 cells were seeded at 2 × 10³/cm².

Human primary osteoblasts were generously provided by Dr Kerry Manton [Manton et al., 2006]. The cells were derived from explants of discarded calvarial bone obtained from a 19-year-old male donor after informed consent according to the ethical guidelines of the Biomedical Research Council of Singapore and the National University Hospital, Singapore. The culture was maintained in DMEM glucose supplemented with 10 % FCS, 2 mM L-glutamine, 100 U/ml penicillin/streptomycin and 100 mg/ml Fungizone.

### Tartrate-resistant Acid Phosphatase Straining

RAW264.7 is a monocyte [Cool et al., 2007] cell line that can be differentiated into tartrate-resistant acid phosphatase (TRAP) positive multinucleated osteoclasts [Collin-Osdoby et al., 2003; Hsu et al., 1999]. As such, they are widely used for the *in vitro* study of osteoclastogenesis. RAW264.7 cells were maintained in alpha-minimum essential medium (α-MEM) containing 10 % FCS, 2 mM glutamine, 100 U/ml penicillin and 100 µg/ml streptomycin in a humidified incubator (5% CO₂ in air) at 37 °C. For experiments, cells were seeded in 8-well chamber slides at a density of 2,000 cells/cm² and cultured for 5 days with 10 ng/ml RANKL (PeproTech, UK) in the presence or absence of 5 µg/ml GAG (soluble or cell surface). The medium and test reagents were refreshed at day 3. At day 5, the adherent cells were fixed with 4 % paraformaldehyde in PBS at 4 °C for 60 min, before being permeablized with 0.2 % Triton X-100 in PBS at room temperature for 5 min. After rinsing with PBS, the cells were then incubated with 0.01 % naphthol AS-MX phosphate and 0.05 % fast red violet LB salt (Sigma, USA) in the presence of 50 mM sodium tartrate and 90 mM sodium acetate (pH 5.0) for TRAP staining. TRAP-positive cells containing ten or more nuclei were considered to be osteoclasts and counted across 10 random fields at 10x magnification (Olympus IX81). All experiments were repeated three times and the average number of osteoclasts per 10 random fields calculated. Photomicrographs of TRAP stained colonies were taken using an Olympus IX81 microscope equipped with a Spot CCD camera and Image pro-plus v2.0) (Olympus, Japan).

### Western Blot Analysis

For Western blot analysis, RAW264.7 cells were washed in PBS twice before being lysed in RIPA buffer (150 mM NaCl, 10 mM Tris pH 7.4, 2 mM EDTA, 0.5 % Nonidet P-40, 0.1 % sodium dodecyl sulfate [SDS], 1 % Triton X-100, protease inhibitor cocktail) at 4 °C for 10 min. The protein concentration was determined using BCA protein assay kit (Pierce Biotechnology, USA) following the manufacturers' instructions. Protein (20 µg) was then denatured and separated by SDS-PAGE. Thereafter, proteins were transferred to nitrocellulose membranes. The membranes were blocked with 5 % nonfat dry milk in PBS with 0.1 % Tween-20 (PBST) for 1 h at room temperature. Blots were then incubated with primary antibody and the corresponding peroxide-conjugated goat anti-mouse / rabbit secondary antibody (Santa Cruz, USA) for 1 h at room temperature with three washes by PBST in between. The primary antibodies used were rabbit polyclonal phospho-p44/42 MAPK (Erk1/2) (Thr202/Tyr204) (Cell Signaling Technology) and mouse monoclonal actin (Chemicon, USA). For reblotting, membranes were stripped at 50 °C for 30 min in stripping buffer (62.5 mM Tris-HCl, pH 6.7, 2 % SDS, and 0.7 % β-mercaptoethanol). Blots were then washed for 30 min with three changes of PBST. Efficiency of stripping was determined by re-exposure of the membranes to ECL. Thereafter, membranes were blocked and immunoblotted for actin to verify the equivalent loading of samples. Immunoreactive bands were visualized by the enhanced chemiluminescence (ECL) kit according to the manufacturer's instructions (Pierce, USA).

### Confocal Laser Scanning Microscopy

Human primary osteoblasts were grown on chamber slides with or without GAGs for 2 days. Thereafter, the cells were rinsed and fixed in 4 % paraformaldehyde for 10 min followed by permeabilization with 0.1 % Triton X-100 for 5 min. After washing, the cells were blocked in 1 % BSA for 30 min before being incubated with rhodamine phalloidin (Invitrogen, USA) for 20 min. Thereafter, the cells were washed and mounted in VECTASHIELD^{®} Mounting Medium with DAPI (Vector Laboratories, USA). Labeled cells were visualized with Zeiss Axio Imager Z1 system (Carl Zeiss Microlmaging, USA). Images were exported in the tagged-information-file format.

### BrdU (5-bromo-2-deoxyuridine) Incorporation Assay

Skeletal balance involves and interplays between osteoblasts and osteoclasts. Both cell types contact one another and are influenced by factors residing in the osteogenic niche. Since the GAGs were isolated from osteoblasts, we next sought to determine whether they influenced osteoblast proliferation. Briefly, primary human osteoblasts seeded at 5000 cells/cm² in 96-well microplate were synchronized by serum starvation for 24 h followed by treatment with GAG for another 24 h. Thereafter, the cells were pulsed with BrdU for 2 h at 37 °C according to the manufacturers' recommednations for BrdU Incorporation Assay kit (Roche, Switzerland). The incorporated BrdU was detected with peroxidase-conjugated anti-BrdU antibody and the bound conjugate visualized with the soluble chromogenic substrate TMB and measured using an ELISA reader (BioRad, USA). The morphology of osteoblasts was also examined by phase-contrast microscopy (Olympus IX81, 10x objective) after 4 days growth in the various conditions.

### RANKL/GAGs Binding Assay

To investigate the RANKL-binding properties to the GAGs extracted in this study, specially-prepared Heparin/GAG binding plates were obtained from Iduron (UK) and an enzyme-based assay performed according to the manufacturer's instructions. The plates were first coated with 5 µg soluble or cell surface GAG overnight in standard assay buffer (100mM NaCl, 50mM sodium acetate, 0.2% Tween 20, pH7.2). After blocking in 0.2 % gelatin, 10 ng RANKL was applied to the plate surface. The GAG-coated wells not subjected to RANKL served as blank controls. Wells not pre-coated with GAGs but subjected to RANKL were used to show that RANKL does not bind to the plate surface. RANKL binding to soluble and cell surface GAG was then detected by its specific antibody conjugated with biotin (Abcam, UK). Biotin was further bound with ExtrAvidin-AP followed by p-nitrophenyl phosphate, which is the chromogenic substrate of AP and gives a yellow-color product detectable at 405 nm using Victor³ multilabel plate reader (PerkinElmer, USA). The whole assay was performed at room temperature and protected from light, with each step followed by extensive washing to remove non-specific interactions. The readings were subtracted by that of the blank control and analyzed.

### Heparin Sepharose Beads Precipitation Assay

RANKL (10 ng) was incubated with 30 µl of a 50 % heparin-Sepharose CL 6B beads slurry (Amersham Pharmacia Biotech, UK) in 100 µl of PBS in the presence or absence of competing heparin (5 µg) or GAG (5 µg) for 20 min at 4 °C. The beads were then washed twice with PBS and the bound RANKL eluted by boiling in Laemmli buffer (Sigma, USA) and detected using anti-RANKL antibody (Santa Cruz, USA) by Western Blot analysis.

### Statistic Analysis

Each experiment was repeated three times and data expressed as means ± SEM. Differences among treatments were analyzed by Students' *t* test or Analysis of Variables (ANOVA). Significant difference was set at *P* < 0.05.

### RESULTS

### Soluble GAGs differed from cell surface GAGs in composition

GAGs are present on cell surfaces, secreted into the extracelluar microenvironment or trapped into the extracelluar matrix (ECM). We therefore chose to isolate and purified GAGs based on their localization from primary porcine osteoblasts. Unfortunately the quantity of ECM GAG was significantly lower than the other two fractions (data not shown) and insufficient for further assays, thus only soluble and cell surface GAGs were applied in this study. Anion exchange chromatography was performed to purify the GAGs. As shown in Figure 1A, GAGs eluted from fraction numbers 640 to 760 and were collected. Since the soluble and cell surface GAGs elute at the same fraction numbers only one representative chromatograph is shown. The conductivity of NaCl also generated a signal as shown in Figure 1A that highlights GAG elution following a step-gradient of 1 M NaCl. We next determined the size distribution of the GAGs to see whether there may be differences in the relative constituents between soluble and cell surface GAGs. We observed two major GAG species (peak 1 and 2) in the soluble GAG and only one major GAG species (peak 2) in the cell surface GAG (Fig. 1B). Chondroitin sulfate (CS) and heparin sulfate (HS) are the two most abundant GAG species in bone (Dynamics of bone and cartilage metabolism, M. J. Seibel, Simon P. Robins, John P. Bilezikian, pp85), with a higher molecular weight of CS compared with HS. It is possible that peak 1 in the soluble GAG fraction is composed of CS and peak 2 of HS however digestion with chondroitin lyase and heparin lyase is necessary to confirm this. Since we were concerned with maintaining a mixture of GAG, as being reflective of the pooled variants osteoclasts are likely to encounter in the bone microenvironment, we chose not to examine this any further.

### Affinity of GAGs for RANKL

To determine whether the soluble and cell surface GAGs bind to RANKL, we immobilized 5 µg of GAG on the surface of heparin/GAG binding plates. Next, RANKL (10-250 ng) was subjected to GAGs pre-bound to the plate surface. Thus RANKL affinity for the GAG could be determined and quantified by ELISA using a biotinylated RANKL antibody. As shown in Figure 2A, irrespective of GAG localization, RANKL binding was observed. Moreover, RANKL binding increased dose-dependently from 10 to 250 ng/ml when 5 µg of GAG was pre-bound to the plate surface, further suggesting that 5 µg/ml of either GAG was sufficient for RANKL binding. Therefore, to examine the affect of GAG on RANKL-induced osteoclastogenesis, 5 µg/ml of either GAG was exogenously applied to RAW264.7 cells, as this was considered sufficient to bind to and interact with 10 ng/ml RANKL. Notably, in comparison to soluble GAGs, the cell surface GAGs showed a lower affinity to RANKL (Fig. 2A).

Heparin has recently been reported to bind to RANKL and inhibit its activity [Ariyoshi et al., 2008], therefore we used Sepharose beads coupled with heparin to precipitate RANKL. These beads are routinely used in laboratories isolating heparin-binding proteins. By adding excess free GAGs as a competing reagent we can further confirm the relative affinity of either GAG to RANKL by observing whether GAGs compete for the binding of RANKL to the heparin-Sepharose beads. As shown in Figure 2B, the presence of free/unbound heparin diminished the precipitated RANKL, demonstrating that RANKL bound to heparin-Sepharose occurs via a specific protein/heparin interaction. Notably, unbound soluble and cell surface GAG prevented the binding of RANKL to heparin-Sepharose, suggesting that these two GAGs directly bind RANKL. Moreover, cell surface GAG showed less competitive binding to RANKL when compared to soluble GAG, suggesting it has a reduced affinity to RANKL, which is consistent with the results from the heparin-binding plate ELISA assay (Fig. 2A). The amount of RANKL bound to the heparin-Sepharose beads was further quantified by densitometry (Fig. 2C).

### GAGs inhibited RANKL induced osteoclast formation

We next examined the effects of soluble and cell surface GAGs on the RANKL-induced osteoclastic differentiation of RAW264.7 cells. As shown in Figure 3A, cells cultured in the absence of RANKL failed to differentiate into TRAP-positive, multinucleated osteoclasts; whereas 5-day treatment with 10 ng/ml RANKL dramatically changed the morphology of RAW264.7 cultures and resulted in the appearance of multinucleated TRAP-positive osteoclast-like cells. Although RANKL produced large multinucleated TRAP-positive osteoclasts, when combined with osteoblast-specific GAGs, the size and number of multinucleated, TRAP-positive cells was greatly reduced (Fig.3A) with a hierarchical inhibitory effect being RANKL + soluble GAGs > RANKL + cell surface GAGs > RANKL alone. Furthermore, the average number of osteoclasts per 10 random fields was 73 ± 11 when cells were treated by RANKL alone (Fig.3B). In the presence of 5µg/ml of either GAG, the number of osteoclasts induced by RANKL was reduced by 69 % (soluble GAGs) and 41 % (cell surface GAGs) respectively (Fig. 3B). Therefore, GAGs produced by osteoblasts, especially the soluble fraction had an inhibitory effect on RANKL-induced osteoclastogenesis.

### GAGs oppose the inhibitory effect of RANKL on ERK activity

Extracellular signal-regulated protein kinase (ERK), also termed as p44/42 MAPK, is a central kinase in the signaling pathway responsible for cell proliferation and survival. ERK is activated upon RANKL stimulation and returns to basal levels by 4 h post RANKL stimulation [Hotokezaka et al., 2002]. This elevated ERK activity is apparently related to the survival of osteoclasts, but not their differentiation, since inhibition of the ERK pathway by PD98059 or U0126 does not suppress osteoclast formation [Matsumoto et al., 2000], but rather increases osteoclastogenesis [Hotokezaka et al., 2002]. Consistent with the notion that ERK negatively regulates osteoclast differentiation, we observed that long-term treatment with RANKL diminished the activity of ERK (Fig. 4). We further examined the effect of soluble GAG on ERK activity in the presence of RANKL, since this GAG fraction was more potent at inhibiting RANKL-induced osteoclastogenesis compared to the cell surface GAG. Interestingly, in the presence of soluble GAGs, ERK activity (suppressed by RANKL) was sustained at non-RANKL stimulated levels (Fig. 4), suggesting that soluble GAG is able to block RANKL-induced decreases in ERK signaling, resulting in a decrease in osteoclastogenesis.

### GAGs increased the proliferation of osteoblasts

Since osteoblast factors generate both autocrine as well as exocrine affects, and are intimately involved in regulating skeletal balance, we sought to also determine whether osteoblast-specific GAGs act to regulate their own growth. We found that both soluble and cell surface GAGs dose-dependently increased osteoblast proliferation (Fig. 5B). Indeed GAGs administered at which doses that significantly inhibit osteoclastogenesis (5 µg/ml), were able to enhance osteoblast proliferation by nearly 2 fold. Notably, neither GAG had an effect on osteoblast morphology (Fig.5A). Moreover, irrespective of treatment, cells exhibited a characteristic spindle-like morphology with numerous filopodia extending from the cells.

### DISCUSSION

Bone growth and remodeling is regulated by cytokines, growth factors and extracellular components, such as GAGs which are associated to the cell surface or localized in the extracellular matrix [Cool and Nurcombe, 2006; Sims and Gooi, 2008]. GAGs interplay with growth factors/cytokines in the microenvironment where osteoblasts and osteoclasts co-reside to orchestrate the process of bone remodeling [Ariyoshi et al., 2008; Jackson et al., 2006; Shinmyouzu et al., 2007; Theoleyre et al., 2006]. RANKL/OPG are key growth factors secreted by osteoblasts to control the activity of osteoclasts and like other growth factors, GAGs present in the microenvironment largely modulate their activity. To more fully elucidate the interplay between osteoblast-derived GAGs and RANKL, we used a homogenous population of monocytes (RAW264.7) which has been extensively studied in osteoclast research. This cell line contains no osteoblast or bone marrow stromal cells which can also be the targets of RANKL. Thus the cellular context is simplified yet remaining responsive to RANKL signaling. Studies on several species of GAGs have revealed controversial functions of those complex sugars [Ariyoshi et al., 2008; Ariyoshi et al., 2005; Irie et al., 2007; Lamoureux et al., 2007; Shinmyouzu et al., 2007; Theoleyre et al., 2006], which is not surprising since GAGs are a mixture of polysaccharides with diverse structures and biological activities. In bone tissue, osteoclasts are surrounded by the whole population of GAGs synthesized by osteoblasts, the master cells to regulate osteoclasts. To understand the overall effect of GAGs on osteoclasts, we did not separate single species of GAGs from the pool; our data for the first time have demonstrated that the net effect of GAGs surrounding osteoclast progenitors is inhibitory to RANKL activity. Moreover, the potency of these GAGs as osteoclast inhibitor (69 % reduction by soluble GAGs) is comparable to the *in vitro* effect of bisphosphonate (75 % reduction by 10⁻⁵ M neridronic acid), a highly effective drug widely used for the prevention of bone resorption and treatment of postmenopausal osteoporosis [Nicolin et al., 2007; Watts et al., 1990].

Our data demonstrates that osteoblast-derived GAGs are able to bind RANKL and to inhibit RANKL-induced osteoclastogenesis. These GAGs are expected to contain a mixture of heparan sulfate, chondroitin sulfate, dermatan sulfate, keratin sulfate and hyaluronic acid [Ecarot-Charrier and Broekhuyse, 1987; Hunter et al., 1983; Ling et al., 2006; Nakamura et al., 2001] but not heparin which is produced in basophils and mast cells [Braunsteiner and Thumb, 1963]. Chondroitin sulfate (CS) has recently been shown to reduce the numbers of TRAP positive multinucleated cells induced by RANKL, indicating that it antagonizes RANKL activity [Ariyoshi et al., 2008]. Likewise, dermatan sulfate also shows a high binding activity to RANKL and is able to prevent interactions between RANKL and RANK [Shinmyouzu et al., 2007]. Hyaluronic acid (HA) meanwhile has no effect on osteoclast differentiation, though low-molecular-weight HA has been shown to enhance osteoclastogenesis via up-regulation of RANK protein levels [Ariyoshi et al., 2005]. Similar to our study, these prior reports also used cultured RAW264.7 cells.

In the present study we show that soluble GAG is more potent than cell surface GAG at antagonizing RANKL. Since an examination of the size distribution of these two GAGs revealed molecular weight differences (presumably due to CS that is absent from cell surface GAG), it might be possible that CS in the soluble GAG variant is able to contribute to the inhibitory effect on RANKL since CS has been shown to inhibits osteoclast formation [Ariyoshi et al., 2008]. However, a more detailed enzymatic examination of the relative GAG samples is needed.

In order to more fully determine the mechanism by which osteoblast-derived GAGs affect osteoclastogenesis, we chose to examine the role they play in mediating RANKL decreased ERK signaling [Hotokezaka et al., 2002]. ERK is a well-known mitogenic kinase essential for cell proliferation and its activity regulates RANKL induced osteoclast formation [Hotokezaka et al., 2002; Lee et al., 2002; Matsumoto et al., 2000]. As such, ERK appears to play a "dual role" during osteoclastogenesis wherein activated ERK drives proliferation and enhance the survival of osteoclasts, whilst also inhibiting osteoclast differentiation [Hotokezaka et al., 2002]. Thus, sustained ERK activation blocks osteoclast differentiation. Not surprisingly then, we show that stimulation of RAW264.7 cells with RANKL, a known pro-osteoclastic differentiation factor, results in down regulation of ERK activity. However (unlike cells treated with RANKL alone), when RAW264.7 cells are treated with both RANKL and GAG, ERK activity remains at levels similar to non-RANKL treat cells, suggesting the inhibitory effect of GAGs on RANKL induced osteoclastogenesis is probably mediated by maintenance of active ERK signaling.

The local communication between bone cells is highly important for the control of bone remodeling and formation [Henriksen et al., 2009]. Osteoblasts play a central role by manufacturing autocrine or paracrine factors to regulate osteoclastogenesis (i.e. by RANKL/OPG) and also their own activity (i.e. FGF2 and BMP2). FGF2 and BMP2, important growth factors for osteoblast growth and differentiation, together with other growth and adhesive factors are controlled by heparin sulphate (HS), an abundant GAG present in the local extracellular microenvironment [Jackson et al., 2006; Takada et al., 2003]. We have found in this study that osteoblast-derived total GAGs promote the proliferation of human osteoblasts (Fig.5B). GAGs exhibit either stimulatory or inhibitory effects on osteogenesis depending on their chemical composition [Haupt et al., 2009; Ling et al., 2006; Manton et al., 2006]. Either soluble or cell surface GAGs are a mixture of several different GAG species which vary from each other in their structure and functions. However, the overall effect of GAGs on human osteoblasts is stimulatory. Together with the ability of these GAGs to inhibit osteoclastogenesis, it suggests that the bone-specific GAGs shift the balance of bone remodeling towards bone formation by favoring osteoblastogenesis while antagonizing osteoclastogenesis.

Collectively, our results demonstrated that in the absence of OPG, the decoy and inhibitory receptor for RANKL, GAGs reduced the osteoclastogenic ability of RANKL in RAW264.7 cells and the potency of the anti-osteoporotic effect is apparently dependent on the composition of the GAGs. The long-term effect of RANKL on ERK is inhibitory, and this corroborates the negative role of ERK on osteoclast differentiation. Also since GAGs recover the level of active ERKs, ERK pathway is apparently an essential pathway in the osteoporotic effect of RANKL. The inhibitory effect of GAGs on osteoclastogenesis suggests an important role of GAGs in bone metabolism and its therapeutic potential in treating osteoporosis.

### References

Anderson DM, Maraskovsky E, Billingsley WL, Dougall WC, Tometsko ME, Roux ER, Teepe MC, DuBose RF, Cosman D, Galibert L. 1997. A homologue of the TNF receptor and its ligand enhance T-cell growth and dendritic-cell function. Nature 390:175-9.
Ariyoshi W, Takahashi T, Kanno T, Ichimiya H, Shinmyouzu K, Takano H, Koseki T, Nishihara T. 2008. Heparin inhibits osteoclastic differentiation and function. J Cell Biochem 103:1707-17.
Ariyoshi W, Takahashi T, Kanno T, Ichimiya H, Takano H, Koseki T, Nishihara T. 2005. Mechanisms involved in enhancement of osteoclast formation and function by low molecular weight hyaluronic acid. J Biol Chem 280:18967-72. Blair JM, Zheng Y, Dunstan CR. 2007. RANK ligand. Int J Biochem Cell Biol 39:1077-81.
Bolon B, Carter C, Daris M, Morony S, Capparelli C, Hsieh A, Mao M, Kostenuik P, Dunstan CR, Lacey DL, Sheng JZ. 2001. Adenoviral delivery of osteoprotegerin ameliorates bone resorption in a mouse ovariectomy model of osteoporosis. Mol Ther 3:197-205.
Boyle WJ, Simonet WS, Lacey DL. 2003. Osteoclast differentiation and activation. Nature 423:337-42.
Braunsteiner H, Thumb N. 1963. [Mast cells, basophils and heparin liberation.]. Bibl Haematol 15:9-15.
Bucay N, Sarosi I, Dunstan CR, Morony S, Tarpley J, Capparelli C, Scully S, Tan HL, Xu W, Lacey DL, Boyle WJ, Simonet WS. 1998. osteoprotegerin-deficient mice develop early onset osteoporosis and arterial calcification. Genes Dev 12:1260-8.
Burgess TL, Qian Y, Kaufman S, Ring BD, Van G, Capparelli C, Kelley M, Hsu H, Boyle WJ, Dunstan CR, Hu S, Lacey DL. 1999. The ligand for osteoprotegerin (OPGL) directly activates mature osteoclasts. J Cell Biol 145:527-38.
Collin-Osdoby P, Yu X, Zheng H, Osdoby P. 2003. RANKL-mediated osteoclast formation from murine RAW 264.7 cells. Methods Mol Med 80:153-66.
Cool SM, Kenny B, Wu A, Nurcombe V, Trau M, Cassady Al, Grondahl L. 2007. Poly(3-hydroxybutyrate-co-3-hydroxyvalerate) composite biomaterials for bone tissue regeneration: in vitro performance assessed by osteoblast proliferation, osteoclast adhesion and resorption, and macrophage proinflammatory response. J Biomed Mater Res A 82:599-610.
Cool SM, Nurcombe V. 2006. Heparan sulfate regulation of progenitor cell fate. J Cell Biochem 99:1040-51.
Dougall WC, Glaccum M, Charrier K, Rohrbach K, Brasel K, De Smedt T, Daro E, Smith J, Tometsko ME, Maliszewski CR, Armstrong A, Shen V, Bain S, Cosman D, Anderson D, Morrissey PJ, Peschon JJ, Schuh J. 1999. RANK is essential for osteoclast and lymph node development. Genes Dev 13:2412-24.
Ecarot-Charrier B, Broekhuyse H. 1987. Proteoglycans synthesized by cultured mouse osteoblasts. J Biol Chem 262:5345-51.
Eghbali-Fatourechi G, Khosla S, Sanyal A, Boyle WJ, Lacey DL, Riggs BL. 2003. Role of RANK ligand in mediating increased bone resorption in early postmenopausal women. J Clin Invest 111:1221-30.
Fuller K, Wong B, Fox S, Choi Y, Chambers TJ. 1998. TRANCE is necessary and sufficient for osteoblast-mediated activation of bone resorption in osteoclasts. J Exp Med 188:997-1001.
Gandhi NS, Mancera RL. 2008. The structure of glycosaminoglycans and their interactions with proteins. Chem Biol Drug Des 72:455-82.
Haupt LM, Murali S, Mun FK, Teplyuk N, Mei LF, Stein GS, van Wijnen AJ, Nurcombe V, Cool SM. 2009. The heparan sulfate proteoglycan (HSPG) glypican-3 mediates commitment of MC3T3-E1 cells toward osteogenesis. J Cell Physiol 220:780-91.
Henriksen K, Neutzsky-Wulff AV, Bonewald LF, Karsdal MA. 2009. Local communication on and within bone controls bone remodeling. Bone 44:1026-33.
Hotokezaka H, Sakai E, Kanaoka K, Saito K, Matsuo K, Kitaura H, Yoshida N, Nakayama K. 2002. U0126 and PD98059, specific inhibitors of MEK, accelerate differentiation of RAW264.7 cells into osteoclast-like cells. J Biol Chem 277:47366-72.
Hsu H, Lacey DL, Dunstan CR, Solovyev I, Colombero A, Timms E, Tan HL, Elliott G, Kelley MJ, Sarosi I, Wang L, Xia XZ, Elliott R, Chiu L, Black T, Scully S, Capparelli C, Morony S, Shimamoto G, Bass MB, Boyle WJ. 1999. Tumor necrosis factor receptor family member RANK mediates osteoclast differentiation and activation induced by osteoprotegerin ligand. Proc Natl Acad Sci U S A 96:3540-5.
Hughes AE, Ralston SH, Marken J, Bell C, MacPherson H, Wallace RG, van Hul W, Whyte MP, Nakatsuka K, Hovy L, Anderson DM. 2000. Mutations in TNFRSF11A, affecting the signal peptide of RANK, cause familial expansile osteolysis. Nat Genet 24:45-8.
Hunter GK, Heersche JN, Aubin JE. 1983. Isolation of three species of proteoglycan synthesized by cloned bone cells. Biochemistry 22:831-7.
lozzo RV. 1998. Matrix proteoglycans: from molecular design to cellular function. Annu Rev Biochem 67:609-52.
Irie A, Takami M, Kubo H, Sekino-Suzuki N, Kasahara K, Sanai Y. 2007. Heparin enhances osteoclastic bone resorption by inhibiting osteoprotegerin activity. Bone 41:165-74.
Jackson RA, Murali S, van Wijnen AJ, Stein GS, Nurcombe V, Cool SM. 2007. Heparan sulfate regulates the anabolic activity of MC3T3-E1 preosteoblast cells by induction of Runx2. J Cell Physiol 210:38-50.
Jackson RA, Nurcombe V, Cool SM. 2006. Coordinated fibroblast growth factor and heparan sulfate regulation of osteogenesis. Gene 379:79-91.
Jimi E, Akiyama S, Tsurukai T, Okahashi N, Kobayashi K, Udagawa N, Nishihara T, Takahashi N, Suda T. 1999. Osteoclast differentiation factor acts as a multifunctional regulator in murine osteoclast differentiation and function. J Immunol 163:434-42.
Karsenty G, Wagner EF. 2002. Reaching a genetic and molecular understanding of skeletal development. Dev Cell 2:389-406.
Katagiri T, Takahashi N. 2002. Regulatory mechanisms of osteoblast and osteoclast differentiation. Oral Dis 8:147-59.
Kim N, Odgren PR, Kim DK, Marks SC, Jr., Choi Y. 2000. Diverse roles of the tumor necrosis factor family member TRANCE in skeletal physiology revealed by TRANCE deficiency and partial rescue by a lymphocyte-expressed TRANCE transgene. Proc Natl Acad Sci U S A 97:10905-10.
Kong YY, Feige U, Sarosi I, Bolon B, Tafuri A, Morony S, Capparelli C, Li J, Elliott R, McCabe S, Wong T, Campagnuolo G, Moran E, Bogoch ER, Van G, Nguyen LT, Ohashi PS, Lacey DL, Fish E, Boyle WJ, Penninger JM. 1999. Activated T cells regulate bone loss and joint destruction in adjuvant arthritis through osteoprotegerin ligand. Nature 402:304-9.
Kumarasuriyar A, Lee I, Nurcombe V, Cool SM. 2009. De-sulfation of MG-63 cell glycosaminoglycans delays in vitro osteogenesis, up-regulates cholesterol synthesis and disrupts cell cycle and the actin cytoskeleton. J Cell Physiol 219:572-83.
Lacey DL, Timms E, Tan HL, Kelley MJ, Dunstan CR, Burgess T, Elliott R, Colombero A, Elliott G, Scully S, Hsu H, Sullivan J, Hawkins N, Davy E, Capparelli C, Eli A, Qian YX, Kaufman S, Sarosi I, Shalhoub V, Senaldi G, Guo J, Delaney J, Boyle WJ. 1998. Osteoprotegerin ligand is a cytokine that regulates osteoclast differentiation and activation. Cell 93:165-76.
Lamoureux F, Baud'huin M, Duplomb L, Heymann D, Redini F. 2007. Proteoglycans: key partners in bone cell biology. Bioessays 29:758-71.
Lee SE, Woo KM, Kim SY, Kim HM, Kwack K, Lee ZH, Kim HH. 2002. The phosphatidylinositol 3-kinase, p38, and extracellular signal-regulated kinase pathways are involved in osteoclast differentiation. Bone 30:71-7.
Li J, Sarosi I, Yan XQ, Morony S, Capparelli C, Tan HL, McCabe S, Elliott R, Scully S, Van G, Kaufman S, Juan SC, Sun Y, Tarpley J, Martin L, Christensen K, McCabe J, Kostenuik P, Hsu H, Fletcher F, Dunstan CR, Lacey DL, Boyle WJ. 2000. RANK is the intrinsic hematopoietic cell surface receptor that controls osteoclastogenesis and regulation of bone mass and calcium metabolism. Proc Natl Acad Sci U S A 97:1566-71.
Ling L, Murali S, Dombrowski C, Haupt LM, Stein GS, van Wijnen AJ, Nurcombe V, Cool SM. 2006. Sulfated glycosaminoglycans mediate the effects of FGF2 on the osteogenic potential of rat calvarial osteoprogenitor cells. J Cell Physiol 209:811-25.
Manton KJ, Sadasivam M, Cool SM, Nurcombe V. 2006. Bone-specific heparan sulfates induce osteoblast growth arrest and downregulation of retinoblastoma protein. J Cell Physiol 209:219-29.
Matsumoto M, Sudo T, Saito T, Osada H, Tsujimoto M. 2000. Involvement of p38 mitogen-activated protein kinase signaling pathway in osteoclastogenesis mediated by receptor activator of NF-kappa B ligand (RANKL). J Biol Chem 275:31155-61.
Matsuzaki K, Udagawa N, Takahashi N, Yamaguchi K, Yasuda H, Shima N, Morinaga T, Toyama Y, Yabe Y, Higashio K, Suda T. 1998. Osteoclast differentiation factor (ODF) induces osteoclast-like cell formation in human peripheral blood mononuclear cell cultures. Biochem Biophys Res Commun 246:199-204.
Mizuno A, Amizuka N, Irie K, Murakami A, Fujise N, Kanno T, Sato Y, Nakagawa N, Yasuda H, Mochizuki S, Gomibuchi T, Yano K, Shima N, Washida N, Tsuda E, Morinaga T, Higashio K, Ozawa H. 1998. Severe osteoporosis in mice lacking osteoclastogenesis inhibitory factor/osteoprotegerin. Biochem Biophys Res Commun 247:610-5.
Nakamura H, Hirata A, Tsuji T, Yamamoto T. 2001. Immunolocalization of keratan sulfate proteoglycan in rat calvaria. Arch Histol Cytol 64:109-18.
Nakatsuka K, Nishizawa Y, Ralston SH. 2003. Phenotypic characterization of early onset Paget's disease of bone caused by a 27-bp duplication in the TNFRSF11A gene. J Bone Miner Res 18:1381-5.
Nicolin V, Bareggi R, Baldini G, Bortul R, Martinelli B, Narducci P. 2007. Effects of neridronic acid on osteoclasts derived by physiological dual-cell cultures. Acta Histochem 109:397-402.
Nurcombe V, Goh FJ, Haupt LM, Murali S, Cool SM. 2007. Temporal and functional changes in glycosaminoglycan expression during osteogenesis. J Mol Histol 38:469-81.
Pearse RN, Sordillo EM, Yaccoby S, Wong BR, Liau DF, Colman N, Michaeli J, Epstein J, Choi Y. 2001. Multiple myeloma disrupts the TRANCE/ osteoprotegerin cytokine axis to trigger bone destruction and promote tumor progression. Proc Natl Acad Sci U S A 98:11581-6.
Perrimon N, Bernfield M. 2000. Specificities of heparan sulphate proteoglycans in developmental processes. Nature 404:725-8.
Roodman GD. 1999. Cell biology of the osteoclast. Exp Hematol 27:1229-41. Sasaki N, Kusano E, Ando Y, Nemoto J, limura O, Ito C, Takeda S, Yano K, Tsuda E, Asano Y. 2002. Changes in osteoprotegerin and markers of bone metabolism during glucocorticoid treatment in patients with chronic glomerulonephritis. Bone 30:853-8.
Schoppet M, Al-Fakhri N, Franke FE, Katz N, Barth PJ, Maisch B, Preissner KT, Hofbauer LC. 2004. Localization of osteoprotegerin, tumor necrosis factor-related apoptosis-inducing ligand, and receptor activator of nuclear factor-kappaB ligand in Monckeberg's sclerosis and atherosclerosis. J Clin Endocrinol Metab 89:4104-12.
Shinmyouzu K, Takahashi T, Ariyoshi W, Ichimiya H, Kanzaki S, Nishihara T. 2007. Dermatan sulfate inhibits osteoclast formation by binding to receptor activator of NF-kappa B ligand. Biochem Biophys Res Commun 354:447-52.
Simonet WS, Lacey DL, Dunstan CR, Kelley M, Chang MS, Luthy R, Nguyen HQ, Wooden S, Bennett L, Boone T, Shimamoto G, DeRose M, Elliott R, Colombero A, Tan HL, Trail G, Sullivan J, Davy E, Bucay N, Renshaw-Gegg L, Hughes TM, Hill D, Pattison W, Campbell P, Sander S, Van G, Tarpley J, Derby P, Lee R, Boyle WJ. 1997. Osteoprotegerin: a novel secreted protein involved in the regulation of bone density. Cell 89:309-19.
Sims NA, Gooi JH. 2008. Bone remodeling: Multiple cellular interactions required for coupling of bone formation and resorption. Semin Cell Dev Biol 19:444-51.
Suda T, Takahashi N, Martin TJ. 1992. Modulation of osteoclast differentiation. Endocr Rev 13:66-80.
Takada T, Katagiri T, Ifuku M, Morimura N, Kobayashi M, Hasegawa K, Ogamo A, Kamijo R. 2003. Sulfated polysaccharides enhance the biological activities of bone morphogenetic proteins. J Biol Chem 278:43229-35.
Theill LE, Boyle WJ, Penninger JM. 2002. RANK-L and RANK: T cells, bone loss, and mammalian evolution. Annu Rev Immunol 20:795-823.
Theoleyre S, Kwan Tat S, Vusio P, Blanchard F, Gallagher J, Ricard-Blum S, Fortun Y, Padrines M, Redini F, Heymann D. 2006. Characterization of osteoprotegerin binding to glycosaminoglycans by surface plasmon resonance: role in the interactions with receptor activator of nuclear factor kappaB ligand (RANKL) and RANK. Biochem Biophys Res Commun 347:460-7.
Watts NB, Harris ST, Genant HK, Wasnich RD, Miller PD, Jackson RD, Licata AA, Ross P, Woodson GC, 3rd, Yanover MJ, et al. 1990. Intermittent cyclical etidronate treatment of postmenopausal osteoporosis. N Engl J Med 323:73-9.
Whyte MP, Hughes AE. 2002. Expansile skeletal hyperphosphatasia is caused by a 15-base pair tandem duplication in TNFRSF11A encoding RANK and is allelic to familial expansile osteolysis. J Bone Miner Res 17:26-9.
Whyte MP, Obrecht SE, Finnegan PM, Jones JL, Podgornik MN, McAlister WH, Mumm S. 2002. Osteoprotegerin deficiency and juvenile Paget's disease. N Engl J Med 347:175-84.
Wong BR, Rho J, Arron J, Robinson E, Orlinick J, Chao M, Kalachikov S, Cayani E, Bartlett FS, 3rd, Frankel WN, Lee SY, Choi Y. 1997. TRANCE is a novel ligand of the tumor necrosis factor receptor family that activates c-Jun N-terminal kinase in T cells. J Biol Chem 272:25190-4.
Yasuda H, Shima N, Nakagawa N, Yamaguchi K, Kinosaki M, Mochizuki S, Tomoyasu A, Yano K, Goto M, Murakami A, Tsuda E, Morinaga T, Higashio K, Udagawa N, Takahashi N, Suda T. 1998. Osteoclast differentiation factor is a ligand for osteoprotegerin/osteoclastogenesis-inhibitory factor and is identical to TRANCE/RANKL. Proc Natl Acad Sci U S A 95:3597-602.

## Claims

1. A soluble glycosaminoglycan mixture obtained from the culture media of a non-confluent cell culture of mammalian osteoblasts for use in the treatment and/or prevention of bone fracture or bone deterioration in a mammal.

2. A soluble glycosaminoglycan mixture obtained from the culture media of a non-confluent cell culture of mammalian osteoblasts for a use according to claim 1 wherein the use comprises administering the glycosaminoglycan mixture to bone tissue of the mammal or to tissue surrounding bone tissue of the mammal.

3. A method of inhibiting osteoclastogenesis, the method comprising administering a soluble glycosaminoglycan mixture obtained from the culture media of a non-confluent cell culture of mammalian osteoblasts to osteoclasts or osteoblasts, *in vitro.*

4. A method of enhancing proliferation of osteoblasts, the method comprising administering a soluble glycosaminoglycan mixture obtained from the culture media of a non-confluent cell culture of mammalian osteoblasts to osteoblasts, *in vitro.*

5. A pharmaceutical composition or medicament, the pharmaceutical composition or medicament comprising a soluble glycosaminoglycan mixture obtained from the culture media of a non-confluent cell culture of mammalian osteoblasts for use in the treatment and/or prevention of bone fracture or bone deterioration in a mammal.

## Patentansprüche

1. Lösliches Glykosaminoglykangemisch, das aus dem Kulturmedium einer nicht konfluenten Zellkultur von Säugetierosteoblasten gewonnen wird, zur Verwendung zur Behandlung und/oder Prävention von Knochenfrakturen oder Knochenabnutzung bei einem Säugetier.

2. Lösliches Glykosaminoglykangemisch, das aus dem Kulturmedium einer nicht konfluenten Zellkultur von Säugetierosteoblasten gewonnen wird, zur Verwendung nach Anspruch 1, wobei die Verwendung das Verabreichen des Glykosaminoglykangemisches an Knochengewebe des Säugetieres oder an das Knochengewebe des Säugetieres umgebendes Gewebe umfasst.

3. Verfahren zur Hemmung von Osteoklastogenese, wobei das Verfahren das Verabreichen eines löslichen Glykosaminoglykangemisches, das aus dem Kulturmedium einer nicht konfluenten Zellkultur von Säugetierosteoblasten gewonnen wird, an Osteoklasten oder Osteoblasten in vitro umfasst.

4. Verfahren zur Verbesserung der Proliferation von Osteoblasten, wobei das Verfahren das Verabreichen eines löslichen Glykosaminoglykangemisches, das aus dem Kulturmedium einer nicht konfluenten Zellkultur von Säugetierosteoblasten gewonnen wird, an Osteoblasten in vitro umfasst.

5. Pharmazeutische Zusammensetzung oder Medikament, wobei die pharmazeutische Zusammensetzung oder das Medikament ein lösliches Glykosaminoglykangemisch umfasst, das aus dem Kulturmedium einer nicht konfluenten Zellkultur von Säugetierosteoblasten gewonnen wird, zur Verwendung zur Behandlung und/oder Prävention von Knochenfrakturen oder Knochenabnutzung bei einem Säugetier.

## Revendications

1. Mélange de glycosaminoglycanes soluble obtenu à partir de milieux de culture d'une culture cellulaire non-confluente d'ostéoblastes mammifères pour une utilisation dans le traitement et/ou la prévention d'une fracture osseuse ou d'une détérioration osseuse chez un mammifère.

2. Mélange de glycosaminoglycanes soluble obtenu à partir de milieux de culture d'une culture cellulaire non-confluente d'ostéoblastes mammifères pour une utilisation selon la revendication 1, dans lequel l'utilisation comprend l'administration du mélange de glycosaminoglycanes dans un tissu osseux du mammifère ou dans un tissu entourant le tissu osseux du mammifère.

3. Procédé d'inhibition d'ostéoclastogenèse, le procédé comprenant l'administration d'un mélange de glycosaminoglycanes soluble obtenu à partir de milieux de culture d'une culture cellulaire non-confluente d'ostéoblastes mammifères à des ostéoclastes ou ostéoblastes, *in vitro.*

4. Procédé d'accentuation de la prolifération d'ostéoblastes, le procédé comprenant l'administration d'un mélange de glycosaminoglycanes soluble obtenu à partir de milieux de culture d'une culture cellulaire non-confluente d'ostéoblastes mammifères à des ostéoblastes, *in vitro.*

5. Composition pharmaceutique ou médicament, la composition pharmaceutique ou le médicament comprenant un mélange de glycosaminoglycanes soluble obtenu à partir de milieux de culture d'une culture cellulaire non-confluente d'ostéoblastes mammifères pour une utilisation dans le traitement et/ou la prévention d'une fracture osseuse ou d'une détérioration osseuse chez un mammifère.
